(19) Europäisches Patentamt
European Patent Office
Office européen des brevets

(11) Veröffentlichungsnummer : **0 072 029**
**B1**

# (12) EUROPÄISCHE PATENTSCHRIFT

(45) Veröffentlichungstag der Patentschrift : **22.10.86**

(21) Anmeldenummer : **82107249.3**

(22) Anmeldetag : **10.08.82**

(51) Int. Cl.⁴ : **C 07 D487/04**, C 07 D223/16, C 07 D513/04, A 61 K 31/55 // (C07D487/04, 249:00, 223:00),(C07D513/04, 281:00, 249:00)

## (54) Triazolobenzazepine, Verfahren und Zwischenprodukte zu deren Herstellung und diese enthaltende Arzneimittel.

(30) Priorität : **12.08.81 CH 5201/81**

(43) Veröffentlichungstag der Anmeldung : **16.02.83 Patentblatt 83/07**

(45) Bekanntmachung des Hinweises auf die Patenterteilung : **22.10.86 Patentblatt 86/43**

(84) Benannte Vertragsstaaten :
**AT BE CH DE FR GB IT LI LU NL SE**

(56) Entgegenhaltungen :
**US-A- 3 856 808**
**CHEMICAL ABSTRACTS, Band 95, Nr. 1, 6. Juli 1981, Seite 678, Spalte 2, Zusammenfassung Nr. 7163a COLUMBUS OHIO (US)**
**CHEMICAL ABSTRACTS, Band 92, Nr. 7, 18. Februar 1980, Seite 670, Spalte 2, Zusammenfassung Nr. 58728j COLUMBUS OHIO (US)**

(73) Patentinhaber : **F. HOFFMANN-LA ROCHE & CO. Aktiengesellschaft**

**CH-4002 Basel (CH)**

(72) Erfinder : **Borer, René**
**Thiersteinerstrasse 20**
**CH-4153 Reinach (CH)**
Erfinder : **Gerecke, Max, Dr.**
**Bruderholzstrasse 47**
**CH-4153 Reinach (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 072 029 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Benzazepine. Im speziellen betrifft sie Triazolobenzazepine der allgemeinen Formel

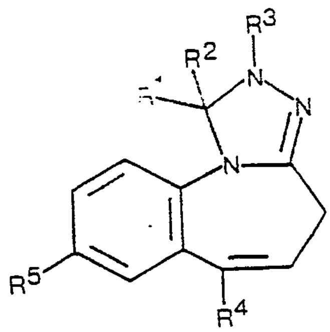

(I)

worin entweder $R^1$ Wasserstoff, niederes Alkyl, 4-Pyridyl oder die Gruppe $-(CH_2)_n-NR^6R^7$ und $R^2$ und $R^3$ zusammen eine zusätzliche Bindung oder $R^1$ und $R^2$ zusammen die Oxogruppe und $R^3$ Wasserstoff oder niederes Alkyl, $R^4$ Phenyl, o-Halogenphenyl oder 2-Pyridyl, $R^5$ Halogen oder Nitro und entweder $R^6$ Wasserstoff oder niederes Alkyl und $R^7$ Wasserstoff, niederes Alkyl, niederes Alkenyl oder niederes Alkinyl oder $R^6$ und $R^7$ zusammen mit dem Stickstoffatom 4-(niederes Alkyl)-1-piperazinyl oder 4-Morpholinyl und n die Zahl 0 oder 1 bedeuten, und pharmazeutisch annehmbare Säureadditionssalze davon.

Diese Verbindungen sind neu und besitzen interessante pharmakologische Eigenschaften.

Gegenstand der vorliegenden Erfindung sind Benzazepine der obigen allgemeinen Formel I und ihre pharmazeutisch annehmbaren Saüreadditionssalze als solche und als pharmazeutische Wirkstoffe, die Herstellung dieser Verbindungen und Zwischenprodukte für die Herstellung dieser Verbindungen, ferner Arzneimittel, enthaltend eine oder mehrere Verbindungen der allgemeinen Formel I oder pharmazeutisch annehnbare Säureadditionssalze davon und die Herstellung solcher Arzneimittel, sowie die Verwendung von Benzazepinen der allgemeinen Formel I und von pharmazeutisch annehmbaren Säureadditionssalzen davon bei der Bekämpfung bzw. Verhütung von Krankheiten.

Der Ausdruck « nieder » in Kombinationen, wie « niederes Alkyl », « niederes Alkenyl », « niederes Alkinyl » und dergleichen, bedeutet, dass die entsprechenden Reste höchstens 7, vorzugsweise höchstens 4, Kohlenstoffatome enthalten. Der Ausdruck « niederes Alkyl » bezeichnet geradkettige oder verzweigte gesättigte Kohlenwasserstoffreste, wie Methyl, Aethyl, n-Propyl, Isopropyl, Isobutyl, n-Butyl, t-Butyl und dergleichen. Der Ausdruck « niederes Alkenyl » umfasst Reste, wie Allyl, Butenyl, Isobutenyl und dergleichen. Der Ausdruck « niederes Alkinyl » umfasst Reste, wie Propargyl und dergleichen. Der Ausdruck « Halogen » bedeutet Fluor, Chlor, Brom oder Jod.

In einer bevorzugten Ausführungsform umfasst die vorliegende Erfindung Verbindungen der obigen Formel I, worin entweder $R^1$ Wasserstoff, Methyl oder Aminomethyl und $R^2$ und $R^3$ zusammen eine zusätzliche Bindung oder $R^1$ und $R^2$ zusammen die Oxogruppe und $R^3$ Wasserstoff bedeuten. $R^4$ bedeutet vorzugsweise o-Chlorphenyl oder o-Fluorphenyl. Die bevorzugte Bedeutungsmöglichkeit von $R^5$ ist Chlor.

Im Rahmen der vorliegenden Erfindung ganz besonders bevorzugte Verbindungen sind :

8-Chlor-6-(2-fluorphenyl)-2,4-dihydro-1H-s-triazolo-[4,3-a] [1] benzazepin-2-on,
1-(Aminomethyl)-8-chlor-6-(2-fluorphenyl)-4H-s-triazolo [4,3-a] [1] benzazepin und
8-Chlor-6-(2-fluorphenyl-1-methyl-4H-s-triazolo-[4,3-a] [1] benzazepin.

Die neuen Verbindungen der obigen Formel I und ihre pharmazeutisch annehmbaren Säureadditionssalze können erfindungsgemäss dadurch hergestellt werden, dass man

a) eine Verbindung der allgemeinen Formel

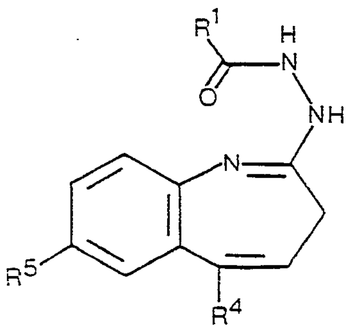

(II) oder

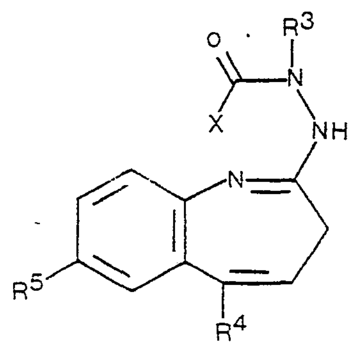

(III)

worin $R^1$, $R^3$, $R^4$ und $R^5$ obige Bedeutung besitzen und X eine Abgangsgruppe bedeutet, cyclisiert, oder

b) eine Verbindung der allgemeinen Formel

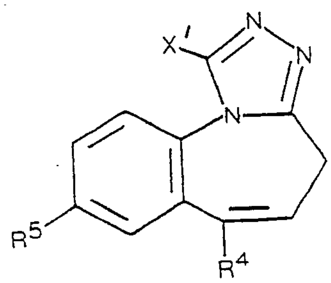

(IV)

worin $R^4$ und $R^5$ obige Bedeutung besitzen und X' eine Abgangsgruppe bedeutet, hydrolysiert, oder

c) eine Verbindung der allgemeinen Formel

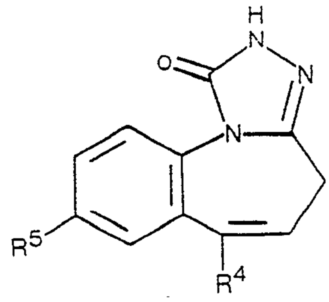

(Ia)

worin $R^4$ und $R^5$ obige Bedeutung besitzen, mit einem einen niederen Alkylrest liefernden Mittel alkyliert, oder

d) eine Verbindung der allgemeinen Formel

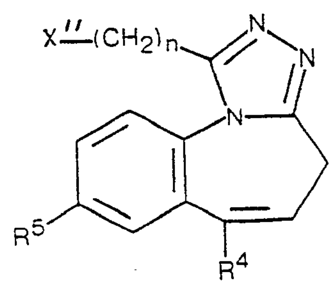

(V)

worin $R^4$, $R^5$ und n obige Bedeutung besitzen und X'' eine Abgangsgruppe bedeutet, mit einem Amin der allgemeinen Formel

$$R^6R^7NH \quad (VI)$$

worin $R^6$ und $R^7$ obige Bedeutung besitzen, umsetzt, oder

e) aus einer Verbindung der allgemeinen Formel

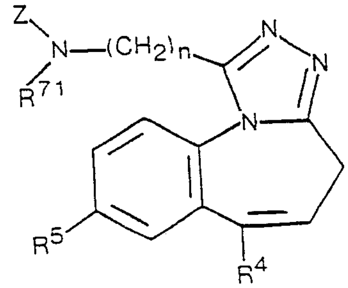

(VII)

worin $R^4$, $R^5$ und n obige Bedeutung besitzen und entweder Z eine Schutzgruppe und $R^{71}$ Wasserstoff, niederes Alkyl, niederes Alkenyl oder niederes Alkinyl oder Z und $R^{71}$ zusammen eine Schutzgruppe bedeuten, die Schutzgruppe abspaltet, oder

f) eine Verbindung der allgemeinen Formel

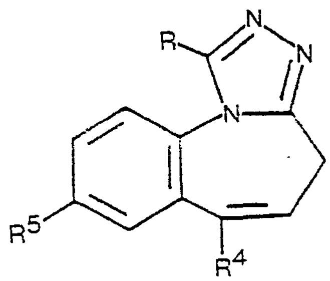

(VIII)

worin R Azido, Azidomethyl, Cyano oder die Gruppe $R^6R^7N—CO—$ bedeutet und $R^4$, $R^5$, $R^6$ und $R^7$ obige Bedeutung besitzen, reduziert, oder

g) eine Verbindung der allgemeinen Formel

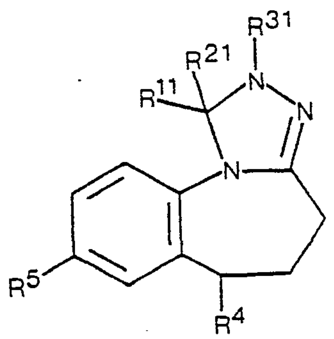

(IX)

worin entweder $R^{11}$ Wasserstoff oder niederes Alkyl und $R^{21}$ und $R^{31}$ zusammen eine zusätzliche Bindung oder $R^{11}$ und $R^{21}$ zusammen die Oxogruppe und $R^{31}$ Wasserstoff oder niederes Alkyl bedeuten und $R^4$ und $R^5$ obige Bedeutung besitzen, dehydriert, oder

h) in einer Verbindung der allgemeinen Formel

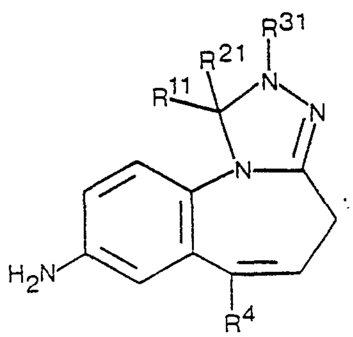

(X)

worin $R^{11}$, $R^{21}$, $R^{31}$ und $R^4$ obige Bedeutung besitzen, die Aminogruppe durch ein Halogenatom oder die Nitrogruppe ersetzt, oder

i) eine Verbindung der allgemeinen Formel

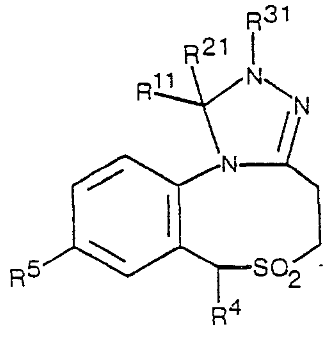

(XI)

worin $R^{11}$, $R^{21}$, $R^{31}$, $R^4$ und $R^5$ obige Bedeutung besitzen, in Gegenwart einer starken Base mit Tetrachlorkohlenstoff und t-Butanol umsetzt, und

j) erwünschtenfalls eine erhaltene Verbindung der Formel I in ein pharmazeutisch annehmbares Säureadditionssalz überführt.

Gemäss Verfahrensvariante a) können Verbindungen der Formel I hergestellt werden, indem man eine Verbindung der Formel II bzw. III cyclisiert. Diese Ringschlussreaktion erfolgt ziemlich leicht und kann gegebenenfalls durch längeres Stehenlassen und/oder durch Anwendung von Wärme bewerkstelligt werden. Man arbeitet zweckmässigerweise in einem inerten organischen Lösungsmittel bei Temperaturen von etwa Raumtemperatur bis etwa 180 °C, vorzugsweise bei der Siedetemperatur des Reaktionsgemisches. Für den vorliegenden Verfahrensaspekt geeignete Lösungsmittel sind z. B. Kohlenwasserstoffe, wie Benzol, Toluol, Xylol und dergleichen, halogenierte Kohlenwasserstoffe, wie Chloroform, Methylenchlorid, Chlorbenzol und dergleichen, Aether, wie Tetrahydrofuran, Dioxan, Diäthylenglykol-dimethyläther, Diäthylenglykol-diäthyläther und dergleichen, Alkohole, wie Methanol, Aethanol, n-Propanol, Isopropanol, n-Butanol, Isobutanol, Cyclohexanol und dergleichen, Hexamethylphosphorsäuretriamid, Dimethylformamid, Dimethylsulfoxid, Essigsäure und dergleichen. Die Verbindungen der Formel II bzw. III müssen nicht notwendigerweise in isoliertem Zustand eingesetzt werden, und in manchen Fällen ist dies auch nicht möglich. In der Regel erweist es sich als zweckmässig, die Verbindungen der Formel II bzw. III ohne Isolierung aus dem Reaktionsgemisch, in dem sie hergestellt worden sind, direkt zu cyclisieren bzw. cyclisieren zu lassen.

Die mit X bezeichnete Abgangsgruppe in einer Verbindung der Formel III ist vorzugsweise ein Chloratom oder ein 1-Imidazolylrest.

Gemäss Verfahrensvariante b) können Verbindungen der Formel I, worin $R^1$ und $R^2$ zusammen die Oxogruppe und $R^3$ Wasserstoff bedeuten, hergestellt werden, indem man eine Verbindung der allgemeinen Formel IV hydrolysiert. Die mit X' bezeichnete Abgangsgruppe in einer Verbindung der Formel IV ist vorzugsweise ein Bromatom oder eine Alkoxy-, Acyloxy- oder Alkylthiogruppe. Die Hydrolyse erfolgt nach an sich bekannten und jedem Fachmann geläufigen Methoden ; je nach verwendeter Abgangsgruppe kann man unter sauren oder basischen Bedingungen arbeiten. Verbindungen der Formel IV, worin X' ein Bromatom oder eine Alkoxy-, Alkylthio- oder Mercaptogruppe bedeutet, können unter sauren Bedingungen hydrolysiert werden, beispielsweise unter Verwendung von konzentrierter Phosphorsäure, Bromwasserstoffsäure, Salzsäure und dergleichen. Verbindungen der Formel IV, worin X' eine Acyloxygruppe bedeutet, werden zweckmässigerweise unter basischen Bedingungen hydrolysiert, beispielsweise unter Verwendung von wässriger Kalilauge, wässriger Natronlauge, wässrigem Kaliumcarbonat und dergleichen. Gegebenenfalls kann man die Reaktion in Gegenwart eines Lösungsvermittlers durchführen ; geeignete Lösungsvermittler sind z. B. Tetrahydrofuran, Dioxan, Dimethylformamid, Alkohole und dergleichen. Verbindungen der Formel IV, worin X' eine schwefelhaltige Abgangsgruppe bedeutet, können aber auch in Gegenwart von Quecksilbersalzen in einem wässrigen System hydrolysiert werden. Die Hydrolyse wird zweckmässigerweise in einem Temperaturbereich von etwa Raumtemperatur bis Siedetemperatur des Reaktionsgemisches durchgeführt.

Gemäss Verfahrensvariante c) können Verbindungen der Formel I, worin $R^1$ und $R^2$ zusammen die Oxogruppe und $R^3$ niederes Alkyl bedeuten, hergestellt werden, indem man eine Verbindung der Formel Ia mit einem einen niederen Alkylrest liefernden Mittel alkyliert. Für den vorliegenden Verfahrensaspekt kann man jedes geeignete Alkylierungsmittel verwenden. Zweckmässigerweise verwendet man dabei Halogenide, wie beispielsweise Methyljodid, Aethyljodid, Isopropylbromid, n-Propylbromid und dergleichen, Dialkylsulfate, wie z. B. Dimethylsulfat und Diäthylsulfat, oder dergleichen und arbeitet in einem inerten organischen Lösungsmittel, beispielsweise in einem Aether, wie z. B. Tetrahydrofuran, Dioxan und Diäthyläther, oder in Aceton, N,N-Dimethylformamid oder dergleichen, in Gegenwart eines säurebindenden Mittels, wie z. B. Kalium- und Natriumcarbonat, zweckmässigerweise bei Raumtemperatur. In einer besonders bevorzugten Ausführungsform verwendet man als Alkylierungsmittel ein Diazoalkan, wie Diazomethan oder Diazoäthan, in einem inerten organischen Lösungsmittel. Geeignete Lösungsmittel sind z. B. Aether, wie Diäthyläther und t-Butylmethyläther oder Mischungen davon mit Alkoholen, wie Methanol und Aethanol, Methylenchlorid, Chloroform oder dergleichen. Vorzugsweise arbeitet man dabei bei Temperaturen unterhalb Raumtemperatur, zweckmässigerweise bei etwa 0 °C.

Gemäss Verfahrensvariante d) können Verbindungen der Formel I, worin $R^1$ die Gruppe $-(CH_2)_n-NR^6R^7$ und $R^2$ und $R^3$ zusammen eine zusätzliche Bindung bedeuten, hergestellt werden, indem man eine Verbindung der Formel V mit einem Amin der Formel VI umsetzt. Die in einer Verbindung der Formel V mit X" bezeichnete Abgangsgruppe umfasst Halogenatome, wie Chlor, Brom und Jod, Sulfonsäurereste, wie Methansulfonyloxy, p-Toluolsulfonyloxy, p-Brombenzolsulfonyloxy und Benzolsulfonyloxy, und andere äquivalente Abgangsgruppen. Diese Reaktion erfolgt zweckmässigerweise in einem inerten organischen Lösungsmittel in Gegenwart eines säurebindenden Mittels. Für den vorliegenden Verfahrensaspekt geeignete Lösungsmittel sind beispielsweise Aether, wie Diäthyläther, t-Butylmethyläther, Tetrahydrofuran, Aethylenglykol-dimethyläther und dergleichen, Alkohole, wie Aethanol, Aethylenglykol und dergleichen, Aceton, Dimethylformamid, Dimethylsulfoxid oder überschüssiges Amin der Formel VI. Als säurebindende Mittel kommen anorganische Basen, wie Kalium- und Natriumcarbonat oder dergleichen, oder organische Basen, wie Triäthylamin, Chinuclidin, Pyridin oder

dergleichen, oder überschüssiges Amin der allgemeinen Formel VI in Frage. Die Reaktionstemperatur kann in einem Bereich von etwa 0 °C bis Siedetemperatur des Reaktionsgemisches variieren und ist natürlich abhängig von der Reaktivität der mit X″ bezeichneten Abgangsgruppe.

Gemäss Verfahrensvariante e) können Verbindungen der Formel I, worin $R^1$ die Gruppe $-(CH_2)_n-NR^6R^7$, $R^2$ und $R^3$ zusammen eine zusätzliche Bindung und $R^6$ Wasserstoff bedeuten, hergestellt werden, indem man aus einer Verbindung der Formel VII die Schutzgruppe abspaltet. Als Schutzgruppen eignen sich für die Zwecke der vorliegenden Erfindung in erster Linie Acylgruppen, vorzugsweise leicht abspaltbare Alkoxycarbonyl- oder Aralkoxycarbonylgruppen, insbesondere die t-Butoxycarbonylgruppe, die Benzyloxycarbonylgruppe usw., ferner auch leicht abspaltbare Aralkylgruppen, wie die Benzylgruppe.

Die Entfernung der Schutzgruppe aus einer Verbindung der Formel VII erfolgt nach an sich bekannten Methoden, wobei natürlich die Natur der zu entfernenden Schutzgruppe fur die Wahl der zur Anwendung gelangenden Methode in Betracht gezogen werden muss. Ebenfalls zu beachten ist natürlich, dass nur solche Methoden verwendet werden können, die die Schutzgruppe selektiv entfernen, ohne das andere im Molekül vorhandene Strukturelement in Mitleidenschaft gezogen werden.

Die weiter oben als Beispiele für in Betracht kommende Schutzgruppen erwähnten Reste können je nach ihrer Natur hydrogenolytisch und/oder hydrolytisch abgespalten werden. So können z. B. die Benzyloxycarbonylgruppe und die t-Butoxycarbonylgruppe unter selektiven sauren Bedingungen abgespalten werden, z. B. durch Behandeln mit einem Gemisch von Bromwasserstoff und Eisessig oder durch Behandeln mit Bortrifluorid oder Bortribromid in einem inerten organischen Lösungsmittel, wie Methylenchlorid. Die t-Butoxycarbonylgruppe kann auch durch Behandeln mit Chlorwasserstoff in einem inerten organischen Lösungsmittel, wie Dioxan, Tetrahydrofuran oder dergleichen, oder durch Behandeln mit Trifluoressigsäure abgespalten werden. Die Benzylgruppe kann durch katalytische Hydrierung, z. B. über Palladium/Kohle entfernt werden. Die Acetylgruppe kann unter milden alkalischen Bedingungen abgespalten werden, z. B. mit einer Lösung eines Natriumalkoholats im entsprechenden Alkohol (wie methanolisches Natriummethylat).

Bedeuten Z und $R^{71}$ in einer Verbindung der Formel VII zusammen eine einzige Schutzgruppe, so kommen in erster Linie cyclische Imide, beispielsweise Phthalimide, in Frage. Eine solche Schutzgruppe lässt sich, z. B. mit Hydrazin, leicht entfernen.

Gemäss Verfahrensvariante f) können Verbindungen der Formel I, worin $R^1$ Amino oder die Gruppe $-CH_2-NR^6R^7$ und $R^2$ und $R^3$ zusammen eine zusätzliche Bindung bedeuten, dadurch hergestellt werden, dass man eine Verbindung der Formel VIII reduziert. Für den vorliegenden Verfahrensaspekt geeignete Reduktionsmittel sind, je nach der Natur der zu reduzierenden Gruppe, z. B. elementarer Wasserstoff in Gegenwart eines Katalysators, wie Paladium/Kohle, Raney-Nickel, Platinoxid und dergleichen, komplexe Metallhydride, wie Lithiumaluminiumhydrid, und dergleichen. Die notwendigen Reaktionsbedingungen können dabei von jedem Fachmann leicht ermittelt werden.

Gemäss Verfahrensvariante g) können Verbindungen der Formel I, worin entweder $R^1$ Wasserstoff oder niederes Alkyl und $R^2$ und $R^3$ zusammen eine zusätzliche Bindung oder $R^1$ und $R^2$ zusammen die Oxogruppe und $R^3$ Wasserstoff oder niederes Alkyl bedeuten, hergestellt werden, indem man eine Verbindung der Formel IX dehydriert. Diese Dehydrierung kann beispielsweise mit einem Oxidationsmittel, wie 2,3-Dichlor-5,6-dicyanbenzochinon, in einem inerten organischen Lösungsmittel, wie Benzol, Toluol und dergleichen, bewerkstelligt werden. Man arbeitet dabei in einem Temperaturbereich von etwa Raumtemperatur bis zur Siedetemperatur des Lösungsmittels.

Die obige Dehydrierung kann aber auch durchgeführt werden, indem man eine Verbindung der Formel IX in einem inerten organischen Lösungsmittel mit einem Bromierungsmittel behandelt. Geeignete Bromierungsmittel sind z. B. N-Bromsuccinimid, N-Bromacetamid und elementares Brom. Bei Verwendung von elementarem Brom arbeitet man zweckmässigerweise in Gegenwart von Licht. Als Lösungsmittel kommen dabei in erster Linie halogenierte Kohlenwasserstoffe, wie z. B. Methylenchlorid, 1,2-Dichloräthan, Chloroform, Tetrachlorkohlenstoff und dergleichen, oder andere inerte organische Lösungsmittel, wie z. B. Acetonitril, Aether, usw., in Frage. Vorzugsweise arbeitet man bei Temperaturen von etwa 0 °C bis zur Siedetemperatur des gewählten Lösungsmittels.

Gemäss Verfahrensvariante h) können Verbindungen der Formel I, worin entweder $R^1$ Wasserstoff oder niederes Alkyl und $R^2$ und $R^3$ zusammen eine zusätzliche Bindung oder $R^1$ und $R^2$ zusammen die Oxogruppe und $R^3$ Wasserstoff oder niederes Alkyl bedeuten, dadurch hergestellt werden, dass man in einer Verbindung der Formel X die Aminogruppe durch ein Halogenatom oder die Nitrogruppe ersetzt. Zweckmässigerweise geht man dabei so vor, dass man die Aminoverbindung der Formel X in ein entsprechendes Diazoniumsalz überführt und dieses, gegebenenfalls ohne vorgängige Isolierung, mit einem Nitrit, wie Natriumnitrit, oder mit einem Halogenid, z. B. mit einem Chlorid oder Bromid, in Gegenwart eines Kupfer(I)salzes umsetzt. Für die Herstellung der entsprechenden Jodide ist die Anwesenheit eines Kupfer(I)salzes nicht erforderlich. Entsprechende Fluoride werden zweckmässigerweise über die entsprechenden Diazonium-tetrafluoroborate hergestellt, beispielsweise durch Bestrahlen mit UV-Licht. Diese Reaktionen werden in wässriger Lösung bei Temperaturen von etwa — 10 °C bis etwa Raumtemperatur durchgeführt.

Eine Aminoverbindung der allgemeinen Formel X kann aber auch durch Oxidation in die entsprechende Nitroverbindung übergeführt werden. Als Oxidationsmittel eignen sich beispielsweise

Persäuren, wie Peressigsäure, Trifluorperessigsäure, m-Chlorperbenzoesäure und Perbenzoesäure, oder dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., oder dergleichen in Frage. In der Regel arbeitet man bei Temperaturen von etwa 0 °C bis etwa Raumtemperatur.

Gemäss Verfahrenvariante i) können Verbindungen der Formel I, worin entweder $R^1$ Wasserstoff oder Methyl und $R^2$ und $R^3$ zusammen eine zusätzliche Bindung oder $R^1$ und $R^2$ zusammen die Oxogruppe und $R^3$ Wasserstoff oder niederes Alkyl bedeuten, dadurch hergestellt werden, dass man eine Verbindung der allgemeinen Formel XI in Gegenwart einer starken Base mit Tetrachlorkohlenstoff und t-Butanol umsetzt. Geeignete starke Basen sind z. B. Alkalimetallhydroxide, wie Natrium- und Kaliumhydroxid, und Alkalimetallalkoholate, wie Kalium-t-butanolat. In einer bevorzugte Ausführungsform arbeitet man in Gegenwart von wenig Wasser. Die Reaktionstemperatur liegt zweckmässigerweise in einem Bereich von etwa Raumtemperatur bis etwa 80 °C.

Gemäss Verfahrensvariante j) können Verbindungen der allgemeinen Formel I erfindungsgemäss in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen dabei sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formeln II und III können ausgehend von Verbindungen der allgemeinen Formel

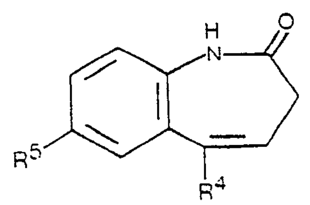

(XII)

worin $R^4$ und $R^5$ obige Bedeutung besitzen, hergestellt werden, und zwar gemäss nachfolgendem Formelschema I, worin $R^1$, $R^3$, $R^4$, $R^5$ und X obige Bedeutung besitzen und X''' eine Abgangsgruppe bedeutet:

Formelschema I

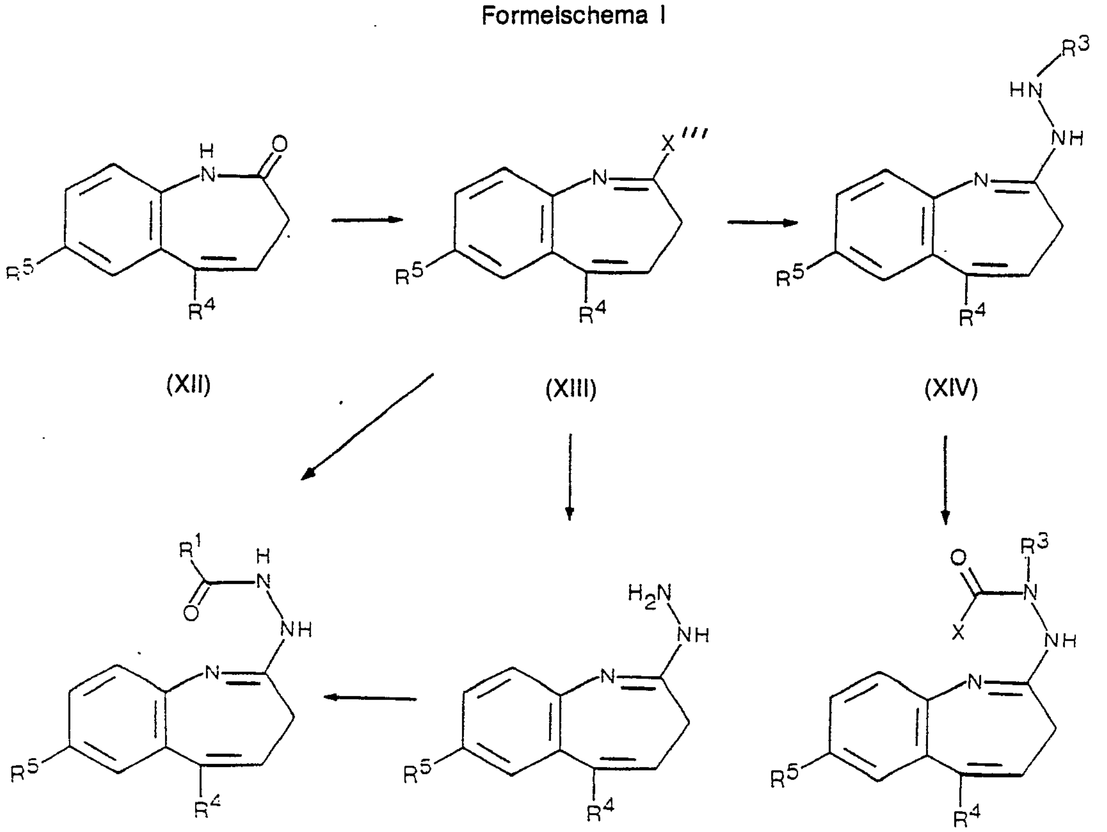

(XII) (XIII) (XIV)

(II) (XIVa) (III)

Die in Formel XII mit X‴ bezeichnete Abgangsgruppe ist beispielsweise eine leicht abspaltbare Phosphinylgruppe, z. B. eine Gruppe der Formel

$$-C\overset{\overset{C}{\|}}{P}(OR^{8})_{2} \quad \text{oder} \quad -O\overset{\overset{O}{\|}}{P}(NR^{9}R^{10})_{2}$$

worin $R^8$ niederes Alkyl und $R^9$ und $R^{10}$ je niederes Alkyl, Allyl, Phenyl oder substituiertes Phenyl oder zusammen mit dem Stickstoffatom einen unsubstituierten oder substituierten heterocyclischen Ring mit 3 bis 8 Gliedern (wie Morpholin) bedeuten, ein Halogenatom, eine Alkylthiogruppe, eine Aralkylthiogruppe, eine N-Nitrosoalkylaminogruppe, eine Alkoxygruppe, eine Mercaptogruppe und dergleichen (wenn X‴ eine Mercaptogruppe bedeutet, so handelt es sich bei der entsprechenden Verbindung der Formel XIII um die Iminothiol-Form des entsprechenden Thiolactams). Die Verbindungen der allgemeinen Formel XIII können nach an sich bekannten Methoden aus Verbindungen der Formel XII hergestellt werden ; vgl. z. B. die Belgischen Patentschriften Nrs. 802 233, 833 249 und 865 653, die Amerikanische Patentschrift Nr. 3 681 341 und J. Org. Chemistry *29*, 231 (1964).

Durch Reaktion einer Verbindung der Formel XIII mit einem Hydrazin der Formel $R^3NH—NH_2$, worin $R^3$ obige Bedeutung besitzt, in einem inerten organischen Lösungsmittel erhält man eine Verbindung der allgemeinen Formel XIV. Geeignete Lösungsmittel sind z. B. Aether, wie Tetrahydrofuran, Dioxan, t-Butylmethyläther, Aethylenglykoldimethyläther und dergleichen, Alkohole, wie Methanol, Aethanol und dergleichen, Aceton, Dimethylformamid, etc. Man arbeitet dabei in einem Temperaturbereich von etwa 0 °C bis zur Siedetemperatur der Reaktionsmischung, jedoch vorzugsweise bei Raumtemperatur.

Durch Reaktion einer Verbindung der Formel XIV mit einem reaktiven Derivat der Kohlensäure erhält man eine Verbindung der Formel III. Geeignete reaktive Derivate der Kohlensäure sind z. B. Phosgen, N,N'-Carbonyldiimidazol und dergleichen. Man arbeitet zweckmässigerweise in einem inerten organischen Lösungsmittel, beispielsweise in einem Aether, wie Tetrahydrofuran, Dioxan, t-Butylmethyläther, Aethylenglykol-dimethyläther und dergleichen, in einem Kohlenwasserstoff, wie Benzol, Toluol, Xylol und dergleichen, in Dimethylformamid, Acetonitril, etc. Verwendet man als reaktives Kohlensäurederivat Phosgen, so arbeitet man vorteilhafterweise in Gegenwart eines säurebindenden Mittels. Besonders bevorzugte säurebindende Mittel sind z. B. tertiäre Amine, wie Pyridin, Triäthylamin, Chinuclidin und dergleichen.

Die Verbindungen der Formel II können hergestellt werden, indem man eine Verbindung der allgemeinen Formel XIVa mit einem reaktiven Derivat einer Carbonsäure der allgemeinen Formel $R^1COOH$, worin $R^1$ obige Bedeutung besitzt, umsetzt. Geeignete reaktionsfähige Carbonsäurederivate sind z. B. die entsprechenden Ester, Orthoester, Anhydride, Halogenide, etc.

Je nach Reaktivität der eingesetzten Carbonsäurederivate eignen sich die folgenden Lösungsmittel : Aether, wie Tetrahydrofuran, Diäthyläther, Dioxan, Aethylenglykoldimethyläther und dergleichen, Alkohole, wie Methanol, Aethanol, n-Butanol, i-Propanol und dergleichen, Dimethylsulfoxid, Dimethylformamid, Acetonitril, etc. Zweckmässigerweise arbeitet man in einem Temperaturbereich von etwa 0 °C bis zum Siedepunkt der Reaktionsmischung.

Die Verbindungen der Formel II können aber auch hergestellt werden, indem man eine Verbindung der Formel XIII mit einer Verbindung der allgemeinen Formel $R^1CO—NH—NH_2$, worin $R^1$ obige Bedeutung besitzt, umsetzt. Man arbeitet dabei zweckmässigerweise in einem inerten organischen Lösungsmittel, wobei Alkohole, wie Methanol, Aethanol, n-Butanol, i-Propanol und dergleichen bevorzugt werden. Die Reaktion wird vorzugsweise in einem Temperaturbereich von etwa Raumtemperatur bis zum Siedepunkt der Reaktionsmischung durchgeführt.

Wie bereits weiter oben erwähnt, ist es nicht notwendig (und in manchen Fällen auch nicht möglich) die Verbindungen der Formeln II und III zu isolieren ; vielmehr erweist es sich in der Regel als zweckmässig, diese Verbindungen ohne Isolierung aus dem Reaktionsgemisch, indem sie hergestellt worden sind, direkt zu cyclisieren bzw. cyclisieren zu lassen.

Die Verbindungen der Formel XII gehören einer an sich bekannten Stoffklasse an. Sie können beispielsweise gemäss dem nachfolgenden Reaktionsschema II, worin $R^4$ und $R^5$ obige Bedeutung besitzen, hergestellt werden :

(Siehe Schema II Seite 9 f.)

Schema II

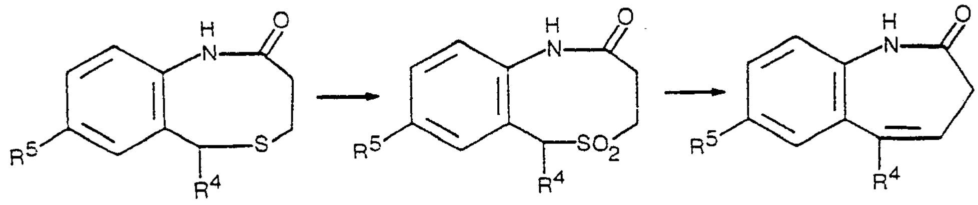

(XV) (XVI) (XII)

Die Oxidation einer Verbindung der allgemeinen Formel XV zu einer Verbindung der allgemeinen Formel XVI erfolgt vorzugsweise mit einem Oxidationsmittel, wie m-Chlorperbenzoesäure oder dergleichen, in einem inerten organischen Lösungsmittel. Geeignete Lösungsmittel sind z. B. halogenierte Kohlenwasserstoffe, wie Methylenchlorid, Chloroform, 1,2-Dichloräthan oder dergleichen. Man arbeitet zweckmässigerweise in einem Temperaturbereich von etwa 0 °C bis zur Siedetemperatur des Reaktionsgemisches, vorzugsweise bei etwa Raumtemperatur.

In Analogie zu Verfahrensvariante i) können die Verbindungen der allgemeinen Formel XVI in Verbindungen der allgemeinen Formel XII übergeführt werden.

Die als Ausgangsstoffe verwendeten Verbindungen der Formel IV, worin X' Brom bedeutet, können z. B. hergestellt werden, indem man eine Verbindung der Formel I, worin $R^1$ Wasserstoff bedeutet, bromiert. Als Bromierungsmittel verwendet man vorzugsweise elementares Brom in einem inerten organischen Lösungsmittel, vorzugsweise in Gegenwart eines säurebindenden Mittels. Geeignete inerte organische Lösungsmittel sind z. B. halogenierte Kohlenwasserstoffe, wie Methylenchlorid, Chloroform, Tetrachlorkohlenstoff und dergleichen, Alkohole, wie Methanol, Aethanol, i-Propanol und dergleichen, Aether, wie Tetrahydrofuran und Dioxan, und dergleichen. Als säurebindendes Mittels verwendet man vorzugsweise ein tertiäres Amin, wie Pyridin, Triäthylamin, Chinuclidin und dergleichen. Die Reaktionstemperatur liegt vorzugsweise in einem Bereich von etwa Raumtemperatur bis etwa 70 °C.

Verbindungen der Formel IV, worin X' niederes Alkoxy, niederes Alkylthio oder Acyloxy bedeutet, können hergestellt werden, indem man eine Verbindung der allgemeinen Formel

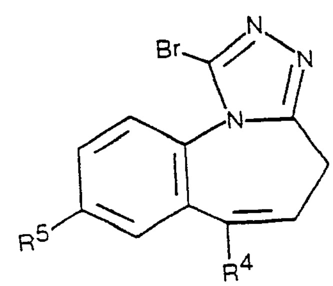

(IVa)

worin $R^4$ und $R^5$ obige Bedeutung besitzen, in Gegenwart einer Base mit einem Alkohol bzw. einem Mercaptan bzw. einer Carbonsäure umsetzt. Geeignete Basen sind z. B. Alkalimetallhydroxide, wie Kaliumhydroxid und Natriumhydroxid, Alkalimetallhydride, wie Natriumhydride, Alkalimetallcarbonate, wie Natriumcarbonat und Kaliumcarbonat, tertiäre Amine, wie Triäthylamin, etc. Die Wahl des geeigneten Lösungsmittels und der geeigneten Reaktionstemperatur bereitet dem Fachmann keine Schwierigkeiten.

Die als Ausgangsstoffe verwendeten Verbindungen der Formeln V und VII können ausgehend von entsprechenden Verbindungen der Formel II hergestellt werden, und zwar in Analogie zu Verfahrensvariante a) und zu den für die Herstellung der entsprechenden Ausgangsstoffe beschriebenen Methoden.

Verbindungen der Formel V, worin n die Zahl 1 bedeutet, können auch ausgehend von Verbindungen der allgemeinen Formel

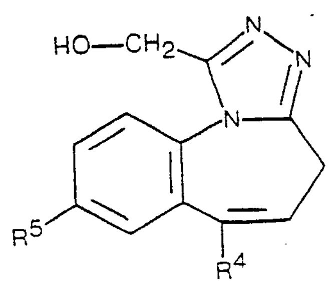

(XVII)

worin $R^4$ und $R^5$ obige Bedeutung besitzen, hergestellt werden. Nach an sich bekannten und jedem Fachmann geläufigen Methoden kann man die Hydroxygruppe in einer Verbindung der Formel XVII durch

eine Abgangsgruppe ersetzen, beispielsweise durch Behandeln einer solchen Verbindung mit einem Halogenierungsmittel, wie Thionylchlorid, Phosphoroxychlorid, Phosphorpentachlorid oder dergleichen, oder durch Behandeln mit einem Sulfonsäurehalogenid, beispielsweise mit Methansulfonsäurechlorid, p-Toluolsulfonsäurechlorid oder dergleichen.

Die Verbindungen der Formel XVII können ausgehend von entsprechenden Verbindungen der Formel II hergestellt werden, und zwar in Analogie zu Verfahrensvariante a) und zu den für die Herstellung der entsprechenden Ausgangsstoffe beschriebenen Methoden.

Verbindungen der Formel VII, worin $R^{71}$ niederes Alkyl, niederes Alkenyl oder niederes Alkinyl bedeutet, können auch hergestellt werden, indem man eine Verbindung der Formel I, worin $R^1$ die Gruppe $-(CH_2)_n-NH_2$ bedeutet, mit einem den Rest Z liefernden Mittel umsetzt und die erhaltene Verbindung der allgemeinen Formel

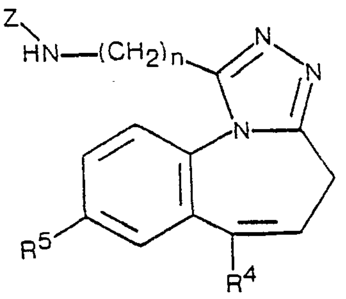

(XVIII)

worin $R^4$, $R^5$, n und Z obige Bedeutung besitzen, entsprechend alkyliert.

Die als Ausgangsstoffe verwendeten Verbindungen der Formel VIII, worin R Azido, Azidomethyl oder Cyano bedeutet, können hergestellt werden, indem man eine Verbindung der Formel V mit einem Azid, wie Natriumazid, bzw. mit einem Cyanid, wie Natriumcyanid oder Kaliumcyanid, umsetzt. Diese Reaktionen können nach an sich bekannten und jedem Fachmann geläufigen Methoden durchgeführt werden.

Verbindungen der Formel VIII, worin R die Gruppe $R^6R^7N-CO-$ bedeutet, können ausgehend von entsprechenden Verbindungen der Formel XII hergestellt werden, und zwar in Analogie zu Verfahrensvariante a) und zu den für die Herstellung der entsprechenden Ausgangsstoffe beschriebenen Methoden.

Die als Ausgangsstoffe verwendeten Verbindungen der Formel IX können in Analogie zur Herstellung von Verbindungen der Formel I aus Verbindungen der Formel XII hergestellt werden, und zwar ausgehend von Verbindungen der allgemeinen Formel

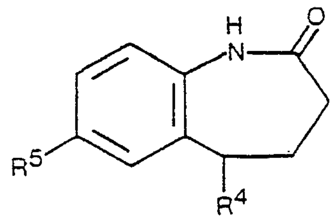

(XIX)

worin $R^4$ und $R^5$ obige Bedeutung besitzen.

Die Verbindungen der Formel XIX gehören einer an sich bekannten Stoffklasse an ; spezifisch nicht vorbeschriebene Vertreter können in Analogie zu den bekannten Vertretern dieser Stoffklasse hergestellt werden.

Die als Ausgangsstoffe verwendeten Verbindungen der Formel X können in Analogie zur Herstellung von Verbindungen der Formel I aus Verbindungen der Formel XII hergestellt werden, und zwar ausgehend von Verbindungen entsprechend der Formel XII, worin aber $R^5$ eine geschützte Aminogruppe bedeutet. Durch Abspalten der mit Z' bezeichneten Schutzgruppe aus einer so erhaltenen Verbindung der allgemeinen Formel

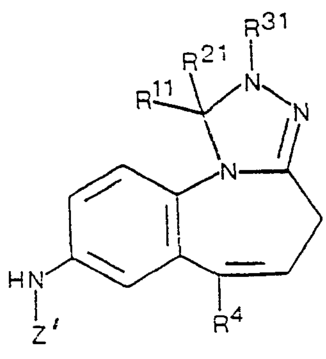

(XX)

worin $R^{11}$, $R^{21}$, $R^{31}$ und $R^4$ obige Bedeutung besitzen und Z' eine Schutzgruppe bedeutet, erhält man eine Verbindung der Formel X. Die mit Z' bezeichnete Schutzgruppe ist vorzugsweise eine Alkoxycarbonylgruppe, z. B. die Aethoxycarbonylgruppe, welche nach an sich bekannten Methoden abgespalten werden kann. Die Verbindungen der Formel X können natürlich auch durch Reduktion entsprechender Nitroverbindungen hergestellt werden.

Die als Ausgangsstoffe verwendeten Verbindungen der Formel XI können ausgehend von Verbindungen der Formel XVI hergestellt werden, und zwar in Analogie zur Herstellung von Verbindungen der Formel I aus Verbindungen der Formel XII.

Die Verbindungen der allgemeinen Formeln II, III, IV, V, VII, VIII, IX, X und XI sind ebenfalls Gegenstand der vorliegenden Erfindung.

Die Verbindungen der allgemeinen Formel I besitzen interessante pharmakologische Eigenschaften und weisen nur eine geringe Toxizität auf. Sie entfalten psychotrope Wirkungen und können als Tranquilizer und/oder Anxiolytica verwendet werden.

In Chem. Abstr. *95,* 7163a (1981), vgl. Collect. Czech. Chem. Commun. *46,* 148-160 (1981), sind gewisse 9-Fluor-6-(4-fluorphenyl)-5,6-dihydro-4H-s-triazolo [4,3-a] [1] benzodiazepine beschrieben, welche schwache antikonvulsive und zentral dämpfende Effekte zeigen.

Einige repräsentative Vertreter der durch die allgemeine Formel I definierten Verbindungsklasse sind in den weiter unten beschriebenen Tests untersucht worden, und es hat sich gezeigt, dass sie in Ratten und Affen ausgeprägte anxiolytische Eigenschaften entfalten. In der nachfolgenden Tabelle sind die erhaltenen Versuchsresultate zusammengestellt. Die Tabelle enthält ausserdem Angaben über die akute Toxizität dieser Verbindungen ($DL_{50}$ bei einmaliger oraler Verabreichung an Mäusen).

(Siehe Tabelle Seite 12 f.)

Es folgt eine kurze Beschreibung der in der obigen Tabelle erwähnten Tierversuche :

Anti-Pentetrazol-Test

Man verabreicht Gruppen von 6 weiblichen Mäusen (19-21 g) die zu testende Verbindung oral und 30 Minuten später eine 0,5-proz. Lösung von Pentetrazol in Kochsalzlösung intravenös (0,375 ml/min.) Man misst die Zeit von Beginn der Infusion bis zum Auftreten tonischer Streckungen der hinteren Gliedmassen und berechnet daraus die bis zu diesem Zeitpunkt verabreichte Dosis Pentetrazol, welche als konvulsive Schwellendosis bezeichnet wird. Die $PR_{2,0}$ bezieht sich auf diejenige Dosis der Testsubstanz, die notwendig ist, damit das Verhältnis konvulsive Schwellendosis in behandelten Tieren zu konvulsiver Schwellendosis in unbehandelten Tieren 2 : 1 beträgt.

Anti-3-Mercaptopropionsäure-Test

Man verabreicht weiblichen Mäusen (19-21 g) die zu testende Verbindung oral und 30 Minuten später 48,8 mg/kg i.p. 3-Mecaptopropionsäure, eine Dosis, welche in unbehandelten Tieren nach 4 Minuten in allen Versuchstieren Emprosthotonus und tonische Streckung der hinteren Gliedmassen bewirkt. Pro Dosis werden 12 Mäuse eingesetzt. Mindestens 6 bis 8 verschiedene Dosen werden getestet. Man bestimmt die Zahl der geschützten Versuchstiere pro Dosisgruppe und ermittelt daraus die $ED_{50}$ mittels Probit-Methode.

Bestimmung der motorischen Aktivität

Gruppen von je drei Ratten (200 g) werden in grossen Käfigen eingeschlossen. Die Versuchstiere können sich während 30 Minuten an die neue Umgebung anpassen ; anschliessend verabreicht man ihnen die Testsubstanz oral. Mittels Infrarot-Lichtschranken werden die spontanen Bewegungen während 2 Stunden automatisch registriert. Als $ED_{40}$ bezeichnet man diejenige Dosis, welche die Beweglichkeit der Versuchstiere im Vergleich zu den unbehandelten Tieren auf 40 % reduziert.

Konflikt-Test an der Ratte

Die Testapparatur ist eine Eintasten-Skinner-Box mit Futterpillengeber. Zur Prüfung potentieller Anxiolytika weden pro Substanz und Dosis in der Regel 8 hungrige Ratten eingesetzt. Man verwendet dabei Ratten, welche auf das bekannte Anxiolytikum Chlordiazepoxid ansprechen. Die Testsubstanzen, welche in einem Gemisch von 10 ml destilliertem Wasser und 2 Tropfen Tween 80 gelöst oder suspendiert sind, werden den Versuchstieren mit Hilfe einer Schlundsonde 30 Minuten vor dem 1-stündigen Konflikttest verabreicht. Während des Versuches, in welchem jeder Tastendruck für eine Futterpille mit einem Fuss-Schock kombiniert ist (Konflikt), wird die Anzahl Tastenbetätigungen registriert. Jedes Versuchstier dient dabei als seine eigene Kontrolle, indem es einmal mit Testsubstanz und einmal mit Kochsalzlösung vorbehandelt wird.

Die erste signifikant anxiolytisch wirksame Dosis (FSD) wird mit dem Wilcoxon-Test (Paarvergleich) bestimmt, indem die Anzahl der Tastenbetätigungen im Hauptversuch (Futterpille + Fuss-Schock, nach

Tabelle

| Verbindung | Toxizität, $DL_{50}$ in mg/kg p.o. | Anti-Pentetrazol-Test, PR 2,0 in mg/kg p.o. | Anti-3-Mercaptopropionsäure-Test, $ED_{50}$ in mg/kg p.o. | Bestimmung der motorischen Aktivität, $ED_{40}$ in mg/kg p.o. | Konflikt-Test an der Ratte, FSD in mg/kg p.o. | Konflikt-Test am Affen in mg/kg p.o. | |
|---|---|---|---|---|---|---|---|
| | | | | | | MED | HED |
| A | > 5000 | 4,1 | 6,0 | >300 | 3 | 1,25 | >80 |
| B | 1000-2000 | 5,8 | 3,7 | >100 | 100 | 1,25 | 10 |
| C | > 5000 | 4,0 | 1,9 | 77 | 3 | 10 | >80 |

Verbindung A : 8-Chlor-6-(2-fluorphenyl)-2,4-dihydro-1H-s-triazolo [4,3-a] [1] benzazepin-1-on ;
Verbindung B : 1-(Aminomethyl)-8-chlor-6-(2-fluorphenyl)-4H-s-triazolo [4,3-a] [1] benzazepin ;
Verbindung C : 8-Chlor-6-(2-fluorphenyl)-1-methyl-4H-s-triazolo [4,3-a] [1] benzazepin.

Vorbehandlung durch Testsubstanz) mit der Anzahl der Tastenbetätigungen im Kontrollversuch (Futterpille + Fuss-Schock, nach Vorbehandlung mit Kochsalzlösung) direkt verglichen wird.

Konflikt-Test an Affen

Als Testapparatur verwendet man eine Zweitasten-Skinner Box mit Futterpillengeber. Als Versuchstiere werden 13 hungrige, 0,8 bis 1 kg schwere, männliche Totenkopf-Affen eingesetzt. Beim Drücken der einen Taste erhalten die Versuchstiere durchschnittlich alle 6 Minuten eine Futterpille. Beim Drücken der anderen Taste erhalten die Versuchstiere viermal häufiger eine Futterpille, im Durchschnitt also alle 1,5 Minuten, werden jedoch gleichzeitig mit einem elektrischen Fuss-Schock bestraft. Der Fuss-Schock bewirkt, dass das Drücken der Taste, welche häufiger Futterpillen abgibt, reduziert wird. Man untersucht nun, ob Testsubstanzen die Tastendrucke, welche mit einem Fuss-Schock kombiniert sind, signifikant erhöhen. Man ermittelt dabei die kleinste (MED) und die höchste signifikant wirksame Dosis (HED).

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen ; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glycerin, pflanzliche Oele und dergleichen. Für Suppositrorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxydantien enthalten. Sie können auch noch andere therapeutisch wertvolle Stoffe enthalten.

Wie eingangs erwähnt sind Arzneimittel, enthaltend eine Verbindung der allgemeinen Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt. Ein weiterer Gegenstand der vorliegenden Erfindung ist — wie eingangs erwähnt — die Verwendung von Verbindungen der allgemeinen Formel I und ihre pharmazeutisch annehmbaren Säureadditionssalze bei der Bekämpfung bzw. Verhütung von Krankheiten, insbesondere bei der Bekämpfung bzw. Verhütung von Angstzuständen. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 5 mg bis 100 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) Zu einer Lösung von 124,8 g 2-Amino-5-chlor-2'-fluorbenzophenon in 1,1 l Aethanol wird innerhalb von 20 Minuten eine Lösung von 18,5 g Natriumborhydrid in 125 ml Wasser bei 20 bis 25° zugetropft. Das Reaktionsgemisch wird während 16 Stunden weitergerührt. Dann gibt man 250 ml Methanol zu und erhitzt 15 Minuten unter Rückfluss zum Sieden. Nach Abdampfen des organischen Lösungsmittels wird der Rückstand mit Wasser verdünnt, worauf man mit Salzsäure ansäuert und mit Essigester extrahiert. Die Essigesterlösungen werden gewaschen, getrocknet, im Vakuum auf 100 ml eingeengt und anschliessend mit 300 ml Petroläther versetzt. Man erhält kristallines 2-Amino-5-chlor-2'-fluorbenzhydrol vom Schmelzpunkt 97-99°.

b) 100 g 2-Amino-5-chlor-2'-fluorbenzhydrol, 45,6 g 3-Mercaptopropionsäure und 200 ml 6N-Salzsäure werden zusammen während 1,5 Stunden bei 100° gerührt. Nach Abkühlen des Reaktionsgemisches werden die ausgefallenen Kristalle abfiltriert und mit 6N-Salzsäure gewaschen. Man erhält 3-[(2-Amino-5-chlorphenyl) (2-fluorphenyl) methylthio] propionsäure-hydrochlorid vom Schmelzpunkt 139-141°.

c) 140 g 3-[2-Amino-5-chlorphenyl) (2-fluorphenyl) methylthio] propionsäure-hydrochlorid werden in 1,4 l Methylenchlorid suspendiert. Die Suspension wird auf 0° gekühlt. Bei dieser Temperatur werden

zunächst 104 ml Triäthylamin zugegeben, worauf innerhalb von 45 Minuten eine Lösung von 37,1 ml Chlorameisensäure-äthylester in 150 ml Methylenchlorid zugetropft wird. Dann wird noch während 3 Stunden bei Raumtemperatur gerührt. Das Reaktionsgemisch wird filtriert. Das Filtrat wird mehrmals mit verdünnter Schwefelsäure und danach mit Wasser gewaschen, getrocknet und eingedampft. Der Rückstand wird in heissem Toluol gelöst, und der unlösliche Anteil wird abfiltriert. Das Filtrat wird stark eingeengt und mit Petroläther versetzt. Es kristallisiert 8-Chlor-6-(2-fluorphenyl)-1,3,4,6-tetrahydro-2H-5,1-benzthiazocin-2-on vom Schmelzpunkt 222° aus.

d) 65 g 8-Chlor-6-(2-fluorphenyl)-1,3,4,6-tetrahydro-2H-5,1-benzthiazocin-2-on werden in 1,95 l Chloroform gelöst. Unter Kühlung wird eine Lösung von 83,2 g m-Chlorperbenzoesäure in 520 ml Chloroform innerhalb von 40 Minuten zugetropft. Die Temperatur wird dabei zwischen 20 und 22° gehalten. Man rührt noch 90 Minuten bei Raumtemperatur weiter und filtriert dann die Reaktionslösung über 800 g Aluminiumoxid (Aktivitätsstufe I). Die mit 2 l Chloroform eluierte Substanz wird aus Methanol/Toluol umkristallisiert. Man erhält 8-Chlor-6-(2-fluorphenyl)-1,3,4,6-tetrahydro-2-oxo-2H-5,1-benzothiazocin-5,5-dioxid vom Schmelzpunkt ca. 265° (Zersetzung).

e) 67,0 g 8-Chlor-6-(2-fluorphenyl)-1,3,4,6-tetrahydro-2-oxo-2H-5,1-benzothiazocin-5,5-dioxid werden in einem Gemisch von 670 ml Tetrachlorkohlenstoff, 670 ml t-Butanol und 31,5 ml Wasser suspendiert. Die Suspension wird auf 35° vorgewärmt. Dann gibt man unter Kühlen rasch vier Portionen von je 51,5 g Kalium-t-butylat hinzu (Reaktionstemperatur beträgt 45-50°). Man rührt noch während 35 Minuten, wobei die Temperatur auf ca. 35° sinkt. Das Reaktionsgemisch wird auf Eis gegossen, worauf man mit Methylenchlorid extrahiert. Die organische Phase wird eingedampft, und der Rückstand wird mit Aether verrührt. Das kristalline Produkt wird abfiltriert. Man erhält 7-Chlor-5-(2-fluorphenyl)-1,3-dihydro-2H-1-benzazepin-2-on vom Schmelzpunkt 214-215°.

f) 20,2 g 7-Chlor-5-(2-fluorphenyl)-1,3-dihydro-2H-1-benzazepin-2-on werden in 110 ml Hexamethylphosphorsäuretriamid gelöst und mit 13,8 g 2,4-Bis-(4-methoxyphenyl)-1,3,2,4-dithiadiphosphetan-2,4-disulfid versetzt. Man erwärmt während 1 Stunde auf 100°, kühlt wieder auf Raumtemperatur und giesst das Reaktionsgemisch in 2,2 l Wasser. Man extrahiert dreimal mit Essigester und wäscht die organischen Auszüge mit Wasser, Natriumhydrogencarbonatlösung und mit gesättigter Natriumchloridlösung. Die Essigesterlösungen werden eingedampft, und der Rückstand wird an 400 g Kieselgel chromatographiert. Durch Eluieren mit Toluol und Toluol/Chloroform (19 : 1) und Umkristallisieren des erhaltenen Stoffes aus Diisopropyläther erhält man 7-Chlor-5-(2-fluorphenyl)-1,3-dihydro-2H-1-benzazepin-2-thion vom Schmelzpunkt 179-181°.

g) 3,9 g 7-Chlor-5-(2-fluorphenyl)-1,3-dihydro-2H-1-benzazepin-2-thion und 4,5 g Essigsäurehydrazid werden in 60 ml n-Butanol während 16 Stunden unter Rückfluss zum Sieden erhitzt. Die Lösung wird dann im Vakuum eingedampft, und der Rückstand wird zwischen Chloroform und Wasser verteilt. Die Chloroformlösung wird eingedampft. Der Rückstand wird an 250 g Kieselgel chromatographiert. Um Verunreinigungen zu entfernen, wird mit einem Toluol/Essigester-Gemisch, mit Essigester und mit Essigester/Aethanol (99 : 1 und 95 : 5) eluiert. Mit Essigester/Aethanol (9 : 1 und 8 : 2) wird rohes 8-Chlor-6-(2-fluorphenyl)-1-methyl-4H-s-triazolo [4,3-a] [1] benzazepin eluiert. Nach Umkristallisieren aus Isopropanol hat das Produkt einen Schmelzpunkt von 224-226°.

Beispiel 2

a) Aus 2-Amino-2',5-dichlorbenzophenon erhält man in Analogie zu Beispiel 1a) 2-Amino-2',5-dichlorbenzhydrol vom Schmelzpunkt 97-100°.

b) Aus 2-Amino-2',5-dichlorbenzhydrol erhält man in Analogie zu Beispiel 1b) 3-[(2-Amino-5-chlorphenyl) (2-chlorphenyl) methylthio] propionsäure-hydrochlorid vom Schmelzpunkt 197° (Zers.).

c) Aus 3-[(2-Amino-5-chlorphenyl) (2-chlorphenyl) methylthio] propionsäure-hydrochlorid erhält man in Analogie zu Beispiel 1c) 8-Chlor-6-(2-chorphenyl)-1,3,4,6-tetrahydro-2H-5,1-benzothiazocin-2-on vom Schmelzpunkt 258-260°.

d) Aus 8-Chlor-6-(2-chlorphenyl)-1,3,4,6-tetrahydro-2H-5,1-benzothiazocin-2-on erhält man in Analogie zu Beispiel 1d) 8-Chlor-6-(2-chlorphenyl)-1,3,4,6-tetrahydro-2-oxo-2H-5,1-benzothiazocin-5,5-dioxid vom Schmelzpunkt 298-300°.

e) Aus 8-Chlor-6-(2-chlorphenyl)-1,3,4,6-tetrahydro-2-oxo-2H-5,1-benzothiazocin-5,5-dioxid erhält man in Analogie zu Beispiel 1e) 7-Chlor-5-(2-chlorphenyl)-1,3-dihydro-2H-1-benzazepin-2-on vom Schmelzpunkt 194-198°.

f) Aus 7-Chlor-5-(2-chlorphenyl)-1,3-dihydro-2H-1-benzazepin-2-on erhält man in Analogie zu Beispiel 1f) 7-Chlor-5-(2-chlorphenyl)-1,3-dihydro-2H-1-benzazepin-2-thion vom Schmelzpunkt 171°.

g) Aus 7-Chlor-5-(2-chlorphenyl)-1,3-dihydro-2H-1-benzazepin-2-thion erhält man in Analogie zu Beispiel 1g) 8-Chlor-6-(2-chlorphenyl)-1-methyl-4H-s-triazolo [4,3-a] [1] benzazepin vom Schmelzpunkt 253-254°.

Beispiel 3

a) Aus 2-(2-Amino-5-brombenzoyl) pyridin erhält man in Analogie zu Beispiel 1a) (2-Amino-5-bromphenyl) (2-pyridyl)-methanol vom Schmelzpunkt 100-104°.

b) Aus (2-Amino-5-bromphenyl) (2-pyridyl) methanol erhält man in Analogie zu Beispiel 1b) 3-[(2-Amino-5-bromphenyl)-(2-pyridyl) methylthio] propionsäure-dihydrochlorid vom Schmelzpunkt 130-132°.

c) Man erhält in Analogie zu Beispiel 1c) 8-Brom-1,3,4,6-tetrahydro-6-(2-pyridyl)-2H-5,1-benzothiazocin-2-on vom Schmelzpunkt 190-191°.

d) Aus 8-Brom-1,3,4,6-tetrahydro-6-(2-pyridyl)-2H-5,1-benzothiazocin-2-on erhält man in Analogie zu Beispiel 1d) 8-Brom-1,3,4,6-tetrahydro-2-oxo-6-(2-pyridyl)-2H-5,1-benzothiazocin-5,5-dioxid vom Schmelzpunkt 215-217°.

e) Aus 8-Brom-1,3,4,6-tetrahydro-2-oxo-6-(2-pyridyl)-2H-5,1-benzothiazocin-5,5-dioxid erhält man in Analogie zu Beispiel 1e) 7-Brom-1,3-dihydro-5-(2-pyridyl)-2H-1-benzazepin-2-on vom Schmelzpunkt 242°.

f) Aus 7-Brom-1,3-dihydro-5-(2-pyridyl)-2H-1-benzazepin-2-on erhält man in Analogie zu Beispiel 1f) 7-Brom-1,3-dihydro-5-(2-pyridyl)-2H-1-benzazepin-2-thion vom Schmelzpunkt 265-267°.

g) Aus 7-Brom-1,3-dihydro-5-(2-pyridyl)-2H-1-benzazepin-2-thion erhält man in Analogie zu Beispiel 1g) 8-Brom-1-methyl-6-(2-pyridyl)-4H-s-triazolo [4,3-a] [1] benzazepin vom Schmelzpunkt 201-202°.

## Beispiel 4

a) Aus 2-Amino-2′-chlor-5-nitrobenzophenon erhält man in Analogie zu Beispiel 1a) 2-Amino-2′-chlor-5-nitrobenzhydrol vom Schmelzpunkt 127-128°.

b) Aus 2-Amino-2′-chlor-5-nitrobenzhydrol erhält man in Analogie zu Beispiel 1b) 3-[(2-Amino-5-nitrophenyl) (2-chlorphenyl) methylthio] propionsäure-hydrochlorid vom Schmelzpunkt 176°.

c) Durch Cyclisieren von 3-[(2-Amino-5-nitrophenyl) (2-chlorphenyl) methylthio] propionsäure-hydrochlorid in Analogie zu Beispiel 1c), jedoch in Tetrahydrofuran und unter Verwendung von Chlorameisensäure-äthylester und Pyridin, erhält man 6-(2-Chlorphenyl)-1,3,4,6-tetrahydro-8-nitro-2H-5,1-benzothiazocin-2-on vom Schmelzpunkt 285°.

d) Aus 6-(2-Chlorphenyl)-1,3,4,6-tetrahydro-8-nitro-2H-5,1-benzothiazocin-2-on erhält man in Analogie zu Beispiel 1d) 6-(2-Chlorphenyl)-1,3,4,6-tetrahydro-8-nitro-2-oxo-5,1-benzothiazocin-5,5-dioxid vom Schmelzpunkt 280°.

e) Aus 6-(2-Chlorphenyl)-1,3,4,6-tetrahydro-8-nitro-2-oxo-5,1-benzothiazocin-5,5-dioxid erhält man in Analogie zu Beispiel 1e) 5-(2-Chlorphenyl)-1,3-dihydro-7-nitro-2H-1-benzazepin-2-on vom Schmelzpunkt 224-226°.

f) 2,36 g 5-(2-Chlorphenyl)-1,3-dihydro-7-nitro-2H-1-benzazepin-2-on und 9,2 ml N,N-Dimethylanilin werden in 100 ml über Aluminiumoxid (Aktivitätsstufe I) filtriertem Chloroform gelöst. Nach Zugabe von 2,8 ml Phosphoroxychlorid wird während 12 Stunden unter Rückfluss zum Sieden erhitzt. Das erhaltene Reaktionsgemisch wird auf eine Lösung von 12 g Natriumhydrogencarbonat in 300 ml Wasser gegossen und während 20 Minuten bei Raumtemperatur gerührt. Die Chloroformlösung wird abgetrennt, gewaschen, getrocknet und im Vakuum eingedampft. Der Rückstand besteht aus rohem 2-Chlor-5-(2-chlorphenyl)-7-nitro-3H-1-benzazepin und Dimethylanilin.

g) Der obige Rückstand wird in 150 ml n-Butanol gelöst, mit 11 g Essigsäurehydrazid versetzt und während 1 Stunde unter Rückfluss zum Sieden erhitzt. Die erhaltene Lösung wird im Vakuum eingedampft ; der Rückstand wird in 150 ml Eisessig gelöst und während 2 Stunden unter Rückfluss zum Sieden erhitzt. Nach Einengen im Vakuum wird der Rückstand zwischen Chloroform und gesättigter Natriumhydrogencarbonatlösung verteilt. Die Chloroformlösung wird im Vakuum eingeengt und der Rückstand wird an 250 g Kieselgel chromatographiert. Zuerst werden Verunreinigungen mit Chloroform/Aethanol (99 : 1 und 98 : 2) eluiert. Das gewünschte Produkt wird mit Chloroform/Aethanol (97 : 3 und 94 : 6) eluiert. Die Rückstände aus diesen Eluaten werden aus Essigester/Aether kristallisiert und dann aus Essigester umkristallisiert. Man erhält 6-(2-Chlorphenyl)-1-methyl-8-nitro-4H-s-triazolo [4,3-a] [1] benzazepin vom Schmelzpunkt 225-227°.

## Beispiel 5

a) Zu einer Lösung von Natriumamid, welche aus 500 ml flüssigem Ammoniak, 0,22 g Eisen (III) nitrat und 13,2 g Natrium zubereitet wurde, tropft man langsam 100 g 2-Fluordiphenylmethan zu. Nach 10 Minuten wird eine Lösung von 40,75 g 3-Brompropionsäure in 500 ml Aether zugetropft. Nach weiteren 15 Minuten lässt man das Ammoniak abdestillieren. Das auskristallisierte Natriumsalz der 4-(2-Fluorphenyl)-4-phenylbuttersäure wird abfiltriert und mit Aether gewaschen. Die freie Säure wird erhalten, indem man das Natriumsalz mit verdünnter Salzsäure versetzt, die Mischung mit Aether extrahiert und die ätherische Lösung eindampft. Man erhält 4-(2-Fluorphenyl)-4-phenylbuttersäure vom Schmelzpunkt 105°.

b) 50 g 4-(2-Fluorphenyl)-4-phenylbuttersäure werden in 250 g Polyphosphorsäure gelöst und unter Rühren während 40 Minuten auf 125° erhitzt. Nach Abkühlen auf Raumtemperatur wird das Gemisch mit Eis vermischt und mit Chloroform extrahiert. Die Chloroformauszüge werden mit Natriumhydrogencarbonatlösung und Wasser neutral gewaschen und dann eingedampft. Das Rohprodukt wird an 500 g Aluminiumoxid (Aktivitätsstufe III) chromatographiert. Durch Eluieren mit Benzol erhält man 4-(2-Fluorphenyl)-1-tetralon.

c) 32 g 4-(2-Fluorphenyl)-1-tetralon in 75 ml Aethanol wird mit einer Lösung von 9,26 g Hydroxylaminhydrochlorid in 10 ml Wasser und dann mit 17 g pulverisiertem Natriumhydroxid versetzt. Man erhitzt

während 5 Minuten unter Rückfluss zum Sieden, kühlt wieder ab, und giesst das Reaktionsgemisch auf ein Gemisch von Eis und verdünnter Salzsäure. Man extrahiert mit Aether und wäscht den erhaltenen Extrakt neutral. Nach Abdampfen des Lösungsmittels erhält man 4-(2-Fluorphenyl)-1-tetralon-oxim vom Schmelzpunkt 120°.

d) 26 g 4-(2-Fluorphenyl)-1-tetralon-oxim werden zu 130 g, auf 125° vorgewärmte Polyphosphorsäure gegeben. Die erhaltene Lösung wird während 30 Minuten bei 125° gerührt, abgekühlt, mit Eis und Wasser vermischt und mit Chloroform extrahiert. Nach Eindampfen der organischen Phase wird der Rückstand aus Aethanol kristallisiert. Man erhält 5-(2-Fluorphenyl)-1,3,4,5-tetrahydro-2H-1-benzazepin-2-on vom Schmelzpunkt 210-212°.

e) Eine Lösung von 17,1 g 5-(2-Fluorphenyl)-1,3,4,5-tetrahydro-2H-1-benzazepin-2-on in 135 ml Dimethylformamid wird mit 9,4 g N-Chlor-succinimid versetzt. Das Gemisch wird während 1 Stunde auf 100° erwärmt, dann abgekühlt und mit Wasser verdünnt. Das ausgefallene Produkt wird mit Essigester extrahiert. Die erhaltene Lösung wird auf ein kleines Volumen eingeengt, wobei 7-Chlor-5-(2-fluorphenyl)-1,3,4,5-tetrahydro-2H-1-benzazepin-2-on vom Schmelzpunkt 185° auskristallisiert.

f) Eine Suspension von 10,25 g 7-Chlor-5-(2-fluorphenyl)-1,3,4,5-tetrahydro-2H-1-benzazepin-2-on in 880 ml Tetrachlorkohlenstoff wird mit 4,5 ml Brom versetzt und während 2 Stunden unter Bestrahlung mit einer 500 W-Glühlampe und unter Rühren zum Sieden erhitzt. Die Lösung wird im Vakuum eingedampft. Der Rückstand wird in 880 ml 2-Propanol gelöst und in Gegenwart von 1,3 g Palladium auf Kohle (5 %) bei Raumtemperatur und Normaldruck hydriert. Der Katalysator wird abfiltriert und das Filtrat eingedampft. Der Rückstand wird in Benzol gelöst und an 295 g Kieselgel chromatographiert. Verunreinigungen und Ausgangsmaterial werden mit Benzol/Essigester (39 : 1) und das gewünschte Produkt mit Benzol/Essigester (19 : 1) eluiert. Nach Umkristallisieren aus Benzol/Aether erhält man 7-Chlor-5-(2-fluorphenyl)-1,3-dihydro-2H-1-benzazepin-2-on vom Schmelzpunkt 214-215°.

g) Eine Lösung von 4 g 7-Chlor-5-(2-fluorphenyl)-1,3-dihydro-2H-1-benzazepin-2-on in 80 ml Methylenchlorid wird mit 10,5 g Triäthyloxonium-tetrafluoroborat versetzt und während 65 Stunden bei Raumtemperatur gerührt. Anschliessend versetzt man mit 12,1 ml einer 50-proz. Kaliumcarbonatlösung. Dann wird Natriumsulfat zugegeben und noch während 30 Minuten gerührt. Die unlöslichen Anteile werden abfiltriert und mit Methylenchlorid gewaschen. Das Filtrat wird eingedampft. Das erhaltene rohe 2-Aethoxy-7-chlor-5-(2-fluorphenyl)-3H-1-benzazepin wird mit einer Lösung von 3,1 g Essigsäurehydrazid in 60 ml n-Butanol versetzt. Das Gemisch wird während 24 Stunden unter Rückfluss zum Sieden erhitzt und dann im Vakuum eingedampft. Nach Verteilen des Rückstandes zwischen Chloroform und Wasser wird die Chloroformlösung im Vakuum eingedampft, und der Rückstand wird aus Aether und dann aus Benzol umkristallisiert. Man erhält 8-Chlor-6-(2-fluorphenyl)-1-methyl-4H-s-triazolo [4,3-a] [1] benzazepin vom Schmelzpunkt 223-225°.

## Beispiel 6

a) Aus 7-Chlor-5-(2-fluorphenyl)-1,3-dihydro-2H-1-benzazepin-2-on und (Benzyloxycarbonylamino)-essigsäurehydrazid erhält man in Analogie zu Beispiel 5g) [[8-Chlor-6-(2-fluorphenyl)-4H-s-triazolo [4,3-a] [1] benzazepin-1-yl] methyl]-carbaminsäure-benzylester vom Schmelzpunkt 161-163°.

b) Eine Lösung von 4,5 g [[8-Chlor-6-(2-fluorphenyl)-4H-s-triazolo [4,3-a] [1] benzazepin-1-yl] methyl]-carbaminsäure-benzylester in 23 ml Eisessig wird bei Raumtemperatur mit 23 ml einer 2,3M-Lösung von Bromwasserstoffsäure in Eisessig versetzt. Man erwärmt während 2 Stunden auf 40°, kühlt auf Raumtemperatur ab und verdünnt mit 200 ml Aether. Das auskristallisierte 1-(Aminomethyl)-8-chlor-6-(2-fluorphenyl)-4H-s-triazolo [4,3-a] [1] benzazepin-hydrobromid wird abfiltriert.

Aus dem obigen Hydrobromid erhält man die freie Base durch Versetzen mit wässrigem Ammoniak und extrahieren mit Chloroform. Der Rückstand aus dem Chloroformextrakt wird aus Benzol/Aether umkristallisiert. Man erhält 1-(Aminomethyl)-8-chlor-6-(2-fluorphenyl)-4H-s-triazolo [4,3-a] [1]-benzazepin vom Schmelzpunkt 188-190°.

Zur Ueberführung in das Hydrochlorid wird die Base in Isopropanol gelöst und mit einer äquivalenten Menge Chlorwasserstoff in Isopropanol behandelt. Nach Abdampfen des Lösungsmittels und Umkristallisieren des Hydrochlorids aus der 10-fachen Menge Wasser erhält man 1-(Aminomethyl)-8-chlor-6-(2-fluorphenyl)-4H-s-triazolo [4,3-a] [1] benzazepin-hydrochlorid vom Schmelzpunkt 246-248° (Zers.).

## Beispiel 7

Aus 7-Chlor-1,3-dihydro-5-phenyl-2H-1-benzazepin-2-on und Essigsäurehydrazid erhält man in Analogie zu Beispiel 5g), und zwar über das 2-Aethoxy-7-chlor-5-phenyl-3H-1-benzazepin, 8-Chlor-1-methyl-6-phenyl-4H-s-triazolo [4,3-a] [1] benzazepin vom Schmelzpunkt 235°.

## Beispiel 8

a) Aus 7-Chlor-5-(2-chlorphenyl)-1,3-dihydro-2H-1-benzazepin-2-on und (Benzyloxycarconylamino)-essigsäurehydrazid erhält man in Analogie zu Beispiel 5g), und zwar über das 2-Aethoxy-7-chlor-5-(2-

chlorphenyl)-3H-1-benzazepin, [[8-Chlor-6-(2-chlorphenyl)-4H-s-triazolo [4,3-a] [1] benzazepin-1-yl] methyl]-carbaminsäure-benzylester vom Schmelzpunkt 190°.

b) Aus [[8-Chlor-6-(2-chlorphenyl)-4H-s-triazolo [4,3-a] [1] benzazepin-1-yl] methyl] carbaminsäure-benzylester erhält man in Analogie zu Beispiel 6b) 1-(Aminomethyl)-8-chlor-6-(2-chlorphenyl)-4H-s-triazolo [4,3-a] [1] benzazepin vom Schmelzpunkt 208-209° und das entsprechende Hydrochlorid vom Schmelzpunkt 256-258° (Zersetzung).

Beispiel 9

Eine Lösung von 1,075 g 7-Chlor-5-phenyl-1,3-dihydro-2H-1-benzazepin-2-on in 20 ml trockenem Tetrahydrofuran wird mit 0,23 g Natriumhydrid (50-proz. Dispersion in Mineralöl) versetzt und während 1 Stunde bei 60° gerührt. Dann kühlt man auf Raumtemperatur ab, versetzt mit 1,53 g Bis-(4-morpholinyl) phosphinsäurechlorid und rührt noch während 2 Stunden bei dieser Temperatur. Anschliessend gibt man eine Lösung von 0,593 g Essigsäurehydrazid in 50 ml n-Butanol dazu, erhitzt während 1 Stunde unter Rückfluss zum Sieden und dampft das Reaktionsgemisch im Vakuum ein. Der Rückstand wird zwischen Wasser und Methylenchlorid verteilt. Die Methylenchloridlösung wird eingedampft und der Rückstand an 100 g Aluminiumoxid (Aktivitätsstufe III) chromatographiert. Verschiedene Verunreinigung und Ausgangsmaterial werden nacheinander mit Benzol und Essigester/Hexan (1 : 1) eluiert. Nach Eluieren mit Chloroform/Aethanol (96 : 4) und Umkristallisieren des Rohproduktes aus Essigester/Benzol/n-Hexan erhält man 8-Chlor-1-methyl-6-phenyl-4H-s-triazolo [4,3-a] [1] benzazepin vom Schmelzpunkt 235°.

Beispiel 10

a) 100 g 2-Amino-2'-chlor-5-nitrobenzophenon werden in 865 ml 25-proz. Salzsäure aufgeschlämmt und portionenweise mit insgesamt 269 g Zinn (II) chlorid versetzt. Das Gemisch wird während 36 Stunden bei Raumtemperatur gerührt, dann mit 0,5 l eines Eis/Wasser-Gemisches verdünnt und mit ca. 2,17 l einer 28-proz. Natriumhydroxidlösung auf pH 10 gestellt. Das Gemisch wird mit Methylenchlorid extrahiert. Nach Abdampfen des Lösungsmittels wird der Rückstand in 0,54 l Aether gelöst und bei ca. —20° kristallisiert. Man erhält 2,5-Diamino-2'-chlorbenzophenon vom Schmelzpunkt 30-40°.

b) Eine Lösung von 91,3 g 2,5-Diamino-2'-chlorbenzophenon in 900 ml Methylenchlorid wird auf —30° abgekühlt und mit 51,6 ml Triäthylamin versetzt. Dann wird über einen Zeitraum von 90 Minuten eine Lösung von 35,2 ml Acetanhydrid in 720 ml Methylenchlorid zugetropft und während 3 Stunden bei —25° und 16 Stunden bei Raumtemperatur stehen gelassen. Man gibt dann 1,4 l Wasser dazu und extrahiert mehrmals mit Methylenchlorid. Die organischen Auszüge werden eingeengt und mit Aether versetzt, wobei 5-Acetamino-2-amino-2'-chlorbenzophenon vom Schmelzpunkt 157° auskristallisiert.

c) Eine Lösung von 108,2 g 5-Acetamino-2-amino-2'-chlorbenzophenon in 1,4 l Aethanol wird bei Raumtemperatur tropfenweise mit einer Lösung von 15,9 g Natriumborhydrid in 100 ml Wasser versetzt. Die erhaltene Lösung wird dann bis zur Entfärbung unter Rückfluss zum Sieden erhitzt und anschliessend, nach Zugabe von 300 ml Methanol, noch weitere 15 Minuten erhitzt. Die organischen Lösungsmittel werden im Vakuum abdestilliert, und der Rückstand wird in 0,5 l Wasser suspendiert. Die erhaltenen Kristalle werden aus Aethanol/Wasser umkristallisiert. Man erhält 5-Acetamino-2-amino-2'-chlorbenzhydrol vom Schmelzpunkt 137-138°.

d) 107,3 g 5-Acetamino-2-amino-2'-chlorbenzhydrol werden zusammen mit 36,8 g 3-Mercaptopropionsäure in 290 ml 6N-Salzsäure während 1 Stunde auf 90° erwärmt. Nach Abkühlen werden die Kristalle abfiltriert. Man erhält 3-[(2,5-Diaminophenyl) (2-chlorphenyl) methylthio] propionsäuredihydrochlorid vom Schmelzpunkt 234-235° (Zersetzung).

e) Eine Lösung von 138,2 g 3-[(2,5-Diaminophenyl) (2-chlorphenyl) methylthio] propionsäure-dihydrochlorid in 8,6 l trockenem Methylenchlorid wird bei —5° mit 94 ml Triäthylamin versetzt. Anschliessend gibt man bei 0 bis 2° nacheinander 129,1 ml Chlorameisensäure-äthylester und 188 ml Triäthylamin hinzu. Dann wird die Kühlung entfernt und während 90 Minuten weitergerührt. Das Reaktionsgemisch wird eingedampft und der Rückstand wird an 1,44 kg Kieselgel chromatographiert. Man eluiert mit Chloroform, um Verunreinigungen zu entfernen, und dann mit Chloroform/Aethanol (89,5 : 1,5). Man erhält 6-(2-Chlorphenyl)-1,3,4,6-tetrahydro-2-oxo-2H-5,1-benzothiazocin-8-carbaminsäure-äthylester vom Schmelzpunkt 229-231°.

f) 64,2 g 6-(2-Chlorphenyl)-1,3,4,6-tetrahydro-2-oxo-2H-5,1-benzothiazocin-8-carbaminsäure-äthylester werden in 3,2 l Chloroform gelöst. Man tropft eine Lösung von 105,3 g m-Chlorperbenzoesäure in 630 ml Chloroform dazu. Nach Abdampfen des Lösungsmittels erhält man 8-(Aethoxycarbonylamino-6-(2-chlorphenyl)-1,3,4,6-tetraydro-2-oxo-2H-5,1-benzothiazocin-5,5-dioxid vom Schmelzpunkt 295-296°.

g) 93,1 g 8-(Aethoxycarbonylamino-6-(2-chlorphenyl)-1,3,4,6-tetrahydro-2-oxo-2H-5,1-benzothiazocin-5,5-dioxid werden bei 80° in einem Gemisch von 1,3 l Tetrachlorkohlenstoff, 1,3 l t-Butanol und 33,5 ml Wasser gelöst. Nach Abkühlen auf 35° versetzt man rasch unter starker Kühlung mit 212 g Kalium-t-butylat (in drei Portionen). Die Temperatur steigt dabei auf 45-50°. Nach Abklingen der Reaktion entfernt man das Kühlbad und rührt noch während 30 Minuten. Das Reaktionsgemisch wird auf Eis gegossen und mehrmals mit Chloroform extrahiert. Die organischen Lösungsmittel werden abgedampft, und der Rückstand wird an 310 g Kieselgel chromatographiert. Man eluiert das gewünschte Produkt mit

Toluol/Chloroform (1 : 3). Nach Umkristallisieren aus Essigester/Petroläther erhält man 5-(2-Chlorphenyl)-2,3-dihydro-2-oxo-1H-1-benzazepin-7-carbaminsäure-äthylester vom Schmelzpunkt 241°.

h) 13,0 g 5-(2-Chlorphenyl)-2,3-dihydro-2-oxo-1H-1-benzazepin-7-carbaminsäure-äthylester werden zusammen mit 39 g Kaliummethylat in einem Gemisch von je 215 ml Methanol, Aethanol und Wasser während 13 Stunden unter Rückfluss zum Sieden erhitzt. Nach Abdampfen der organischen Lösungsmittel extrahiert man den Rückstand mit Chloroform. Der Rückstand aus dem Chloroform-Extrakt wird aus Essigester kristallisiert. Man erhält 7-Amino-5-(2-chlorphenyl)-1,3-dihydro-2H-1-benzazepin-2-on vom Schmelzpunkt 192°.

i) Eine Lösung von 9,2 g 7-Amino-5-(2-chlorphenyl)-1,3-dihydro-2H-1-benzazepin-2-on in 230 ml Methylenchlorid wird bei 0° mit einer Lösung von 22,3 g m-Chlorperbenzoesäure in 190 ml Methylenchlorid versetzt. Die Temperatur steigt dabei bis auf 10°. Man kühlt wieder auf 0° ab, rührt während 2 Minuten und extrahiert dann mit wässeriger Natriumhydrogencarbonatlösung. Die organische Phase wird dann durch basisches Aluminiumoxid filtriert und, nach Prüfung auf Peroxid-Freiheit, eingedampft. Der Rückstand wird in Toluol aufgenommen und an 110 g Kieselgel chromatographiert. Mit Toluol/Chloroform (4 : 1) eluiert man 5-(2-Chlorphenyl)-1,3-dihydro-7-nitro-2H-1-benzazepin-2-on, das nach Umkristallisieren aus Diäthyläther/Diisopropyläther einen Schmelzpunkt von 224-226° aufweist.

j) 2,36 g 5-(2-Chlorphenyl)-1,3-dihydro-7-nitro-2H-1-benzazepin-2-on und 9,2 ml N,N-Dimethylanilin werden in 100 ml über Aluminiumoxid (Aktivitätsstufe I) filtriertem Chloroform gelöst. Nach Zugabe von 2,8 ml Phosphoroxychlorid wird während 12 Stunden unter Rückfluss zum Sieden erhitzt. Das erhaltene Reaktionsgemisch wird auf eine Lösung von 12 g Natriumhydrogencarbonat in 300 ml Wasser gegossen und während 20 Minuten bei Raumtemperatur gerührt. Die Chloroformlösung wird abgetrennt, gewaschen, getrocknet und im Vakuum eingedampft. Der Rückstand besteht aus rohem 2-Chlor-5-(2-chlorphenyl)-7-nitro-3H-1-benzazepin und Dimethylanilin.

k) Der obige Rückstand wird in 150 ml n-Butanol gelöst, mit 11 g Essigsäurehydrazid versetzt und während 1 Stunde unter Rückfluss zum Sieden erhitzt. Die erhaltene Lösung wird im Vakuum eingedampft ; der Rückstand wird in 150 ml Eisessig gelöst und während 2 Stunden unter Rückfluss zum Sieden erhitzt. Nach Einengen im Vakuum wird der Rückstand zwischen Chloroform und gesättigter Natriumhydrogencarbonatlösung verteilt. Die Chloroformlösung wird im Vakuum eingeengt und der Rückstand wird an 250 g Kieselgel chromatographiert. Zuerst werden Verunreinigungen mit Chloroform/Aethanol (99 : 1 und 98 : 2) eluiert. Das gewünschte Produkt wird mit Chloroform/Aethanol (97 : 3 und 94 : 6) eluiert. Die Rückstände aus diesen Eluaten werden aus Essigester/Aether kristallisiert und dann aus Essigester umkristallisiert. Man erhält 6-(2-Chlorphenyl)-1-methyl-8-nitro-4H-s-triazolo [4,3-a] [1] benzazepin vom Schmelzpunkt 225-227°.

## Beispiel 11

a) Aus 2-Chlor-5-(2-chlorphenyl)-7-nitro-3H-1-benzazepin und (Benzyloxycarbonylamino) essigsäurehydrazid erhält man in Analogie zu Beispiel 10k) [[6-(2-Chlorphenyl)-8-nitro-4H-s-triazolo [4,3-a] [1] benzazepin-1-yl] methyl] carbaminsäure-benzylester vom Schmelzpunkt 194-195°.

b) Aus [[6-(2-Chlorphenyl)-8-nitro-4H-s-triazolo [4,3-a] [1] benzazepin-1-yl] methyl] carbaminsäure-benzylester erhält man in Analogie zu Beispiel 6b) 1-(Aminomethyl)-6-(2-chlorphenyl)-8-nitro-4H-s-triazolo [4,3-a] [1] benzazepin vom Schmelzpunkt 206-208°, das entsprechende Hydrobromid vom Schmelzpunkt 202-204° (Zers.) und das entsprechende Hydrochlorid vom Schmelzpunkt 262-263° (Zers.).

## Beispiel 12

6 g 7-Chlor-5-(2-fluorphenyl)-1,3-dihydro-2H-1-benzazepin-2-thion und 27 g Isonicotinsäurehydrazid werden zusammen in 360 ml n-Butanol gelöst und während 24 Stunden unter Rückfluss zum Sieden erhitzt. Nach Eindampfen der erhaltenen Lösung im Vakuum wird der Rückstand zwischen Toluol und Wasser verteilt. Die Toluolphase wird auf ca. 100 ml eingeengt. Die ausgefallenen Kristalle werden abfiltriert und aus Benzol umkristallisiert. Man erhält 8-Chlor-6-(2-fluorphenyl)-1-(4-pyridyl)-4H-s-triazolo-[4,3-a] [1] benzazepin vom Schmelzpunkt 198-200°.

## Beispiel 13

Aus 8-Brom-1,3-dihydro-5-(2-pyridyl)-2H-1-benzazepin-2-thion und Isonicotinsäurehydrazid erhält man in Analogie zu Beispiel 12 8-Brom-6-(2-pyridyl)-1-(4-pyridyl)-4H-s-triazolo [4,3-a] [1] benzazepin vom Schmelzpunkt 267-269°.

## Beispiel 14

Aus 7-Chlor-5-(2-fluorphenyl)-1,3-dihydro-2H-1-benzazepin-2-thion und Dimethylaminoessigsäurehydrazid erhält man in Analogie zu Beispiel 12 8-Chlor-6-(2-fluorphenyl)-1-(dimethylaminomethyl)-4H-s-triazolo [4,3-a] [1] benzazepin vom Schmelzpunkt 196-197°.

Beispiel 15

a) Aus 8-Brom-1,3-dihydro-5-(2-pyridyl)-2H-1-benzazepin-2-thion und (Benzyloxycarbonylamino) essigsäurehydrazid erhält man in Analogie zu Beispiel 12 [[8-Brom-6-(2-pyridyl)-4H-s-triazolo [4,3-a] [1] benzazepin-1-yl] methyl]-carbaminsäure-benzylester vom Schmelzpunkt 128-132°.

b) Aus [[8-Brom-6-(2-pyridyl)-4H-s-triazolo [4,3-a] [1] benzazepin-1-yl] methyl] carbaminsäure-benzylester erhält man in Analogie zu Beispiel 6b) 1-(Aminomethyl)-8-brom-6-(2-pyridyl)-4H-s-triazolo [4,3-a] [1] benzazepin und das entsprechende Maleat vom Schmelzpunkt 165° (Zers.).

Beispiel 16

a) Eine Lösung von 10 ml Hydrazin-hydrat in 1 l Tetrahydrofuran wird mit 12,5 g 7-Chlor-5-(2-fluorphenyl)-1,3-dihydro-2H-1-benzazepin-2-thion versetzt und während 2 Stunden bei Raumtemperatur unter einem Stickstoffstrom gerührt. Das Reaktionsgemisch wird im Vakuum eingedampft. Der Rückstand wird aus Diisopropyläther/Hexan kristallisiert, wobei man 7-Chlor-5-(2-fluorphenyl)-2-hydrazino-3H-1-benzazepin vom Schmelzpunkt 239-240° erhält.

b) 12,1 g 7-Chlor-5-(2-fluorphenyl)-2-hydrazino-3H-1-benzazepin werden in einem Gemisch von 330 ml n-Butanol und 110 ml Orthoameisensäure-triäthylester während 2 Stunden unter Rückfluss zum Sieden erhitzt. Das Reaktionsgemisch wird im Vakuum eingedampft. Der Rückstand wird aus Hexan kristallisiert, wobei man rohes 8-Chlor-6-(2-fluorphenyl)-4H-s-triazolo [4,3-a] [1] benzazepin als rötlich gefärbte Kristalle erhält. Zur Reinigung wird dieses Material an 121 g Kieselgel chromatographiert. Verunreinigungen werden mit Benzol/Methanol (99 : 1) eluiert. Anschliessend eluiert man das Produkt mit Benzol/Methanol (98,5 : 1,5 und 98 : 2). Nach Kristallisieren des erhaltenen Materials aus Aether erhält man 8-Chlor-6-(2-fluorphenyl)-4H-s-triazolo-[4,3-a] [1] benzazepin vom Schmelzpunkt 180-182°.

Beispiel 17

a) In Analogie zu Beispiel 16a) erhält man aus 7-Chlor-5-(2-chlorphenyl)-1,3-dihydro-2H-1-benzazepin-2-thion und Hydrazin-hydrat das 7-Chlor-5-(2-chlorphenyl)-2-hydrazino-3H-1-benzazepin vom Schmelzpunkt 223-225°.

b) In Analogie zu Beispiel 16b) erhält man aus 7-Chlor-5-(2-chlorphenyl)-2-hydrazino-3H-1-benzazepin durch Umsetzen mit Orthoameisensäure-triäthylester das 8-Chlor-6-(2-chlorphenyl)-4H-s-triazolo [4,3-a] [1] benzazepin vom Schmelzpunkt 189-190°.

Beispiel 18

a) In Analogie zu Beispiel 16a) erhält man aus 7-Brom-1,3-dihydro-5-(2-pyridyl)-2H-1-benzazepin-2-thion und Hydrazin-hydrat das 7-Brom-2-hydrazino-5-(2-pyridyl)-3H-1-benzazepin vom Schmelzpunkt 149° (Zers.).

b) In Analogie zu Beispiel 16b) erhält man aus 7-Brom-2-hydrazino-5-(2-pyridyl)-3H-1-benzazepin durch Umsetzen mit Orthoameisensäure-triäthylester das 8-Brom-6-(2-pyridyl)-4H-s-triazolo [4,3-a] [1] benzazepin vom Schmelzpunkt 219°.

Beispiel 19

Aus 7-Chlor-5-(2-fluorphenyl)-2-hydrazino-3H-1-benzazepin und Orthopropionsäure-triäthylester erhält man in Analogie zu Beispiel 16b) das 1-Aethyl-8-chlor-6-(2-fluorphenyl)-4H-s-triazolo [4,3-a] [1] benzazepin vom Schmelzpunkt 238-239°.

Beispiel 20

a) 8,5 g 8-Chloro-6-(2-fluorphenyl)-4H-s-triazolo [4,3-a] [1] benzazepin werden in 135 ml Chloroform und 3,3 ml Pyridin gelöst. Bei Raumtemperatur werden unter Rühren 2,0 ml Brom zugetropft. Man erwärmt dann während 50 Minuten auf 55-60° und dampft im Vakuum ein. Der Rückstand wird an 550 g Kielselgel chromatographiert. Durch Eluieren mit Chloroform, das 0,3 bzw. 0,4 % Methanol enthält, und Umkristallisieren des erhaltenen Materials aus Benzol erhält man 1-Brom-8-chlor-6-(2-fluorphenyl)-4H-s-triazolo [4,3-a] [1] benzazepin vom Schmelzpunkt 222°.

b) 3,9 g 1-Brom-8-chlor-6-(2-fluorphenyl)-4H-s-triazolo [4,3-a] [1] benzazepin werden in 90 ml Methanol suspendiert und mit 1,62 g Natriummethylat versetzt. Das Gemisch wird während 20 Stunden unter Rückfluss zum Sieden erhitzt und dann eingedampft. Der Rückstand, welcher vorwiegend 8-Chlor-6-(2-fluorphenyl)-1-methoxy-4H-s-triazolo [4,3-a] [1] benzazepin enthält, wird in 100 ml 48-proz. Bromwasserstoffsäure während 4,5 Stunden unter Rückfluss zum Sieden erhitzt. Das Reaktionsgemisch wird mit Wasser verdünnt, mit Natriumcarbonat schwach alkalisch gestellt und mit Methylenchlorid extrahiert. Die Methylenchloridlösung wird eingedampft, und der Rückstand wird an 120 g Aluminiumoxid (Aktivitätsstufe III) chromatographiert. Verunreinigungen werden durch Eluieren mit Benzol/Chloroform

(9 : 1 bis 1 : 4) abgetrennt. Durch Eluieren mit Chloroform, das 1 bis 10 % Methanol enthält, und zweimaligem Umkristallisieren des erhaltenen Materials aus Benzol erhält man 8-Chlor-6-(2-fluorphenyl)-2,4-dihydro-1H-s-triazolo [4,3-a] [1] benzazepin-1-on vom Schmelzpunkt 223-225°.

## Beispiel 21

a) 8,1 g 8-Chlor-6-(2-chlorphenyl)-4H-s-triazolo [4,3-a] [1] benzazepin werden nach dem im Beispiel 20a) beschriebenen Verfahren bromiert. Man erhält 1-Brom-8-chlor-6-(2-chlorphenyl)-4H-s-triazolo [4,3-a] [1] benzazepin vom Schmelzpunkt 234°.

b) 9 g 1-Brom-8-chlor-6-(2-chlorphenyl)-4H-s-triazolo-[4,3-a] [1] benzazepin werden in 263 ml 85-proz. Phosphorsäure während 22 Stunden bei 140° gerührt. Das Reaktionsgemisch wird auf 1,5 kg eines Eis/Wasser-Gemisches gegossen und mit 28-proz. Natriumhydroxidlösung neutralisiert. Das Produkt wird mit Chloroform extrahiert. Der Rückstand aus den Chloroformauszügen wird an 300 g Kieselgel chromatographiert. Verunreinigungen werden durch Eluieren mit Chloroform abgetrennt. Durch Eluieren mit Chloroform, das 2 bzw. 4 % Aethanol enthält, Kristallisieren des erhaltenen Materials aus Diäthyläther/Diisopropyläther und Umkristallisieren aus Essigester erhält man 8-Chlor-6-(2-chlorphenyl)-2,4-dihydro-1H-s-triazolo [4,3-a] [1] benzazepin-1-on vom Schmelzpunkt 250-251°.

## Beispiel 22

Aus 1-Brom-8-chlor-6-(2-fluorphenyl)-4H-s-triazolo-[4,3-a] [1] benzazepin erhält man in Analogie zu Beispiel 21b) 8-Chlor-6-(2-fluorphenyl)-2,4-dihydro-1H-s-triazolo [4,3-a] [1] benzazepin-1-on vom Schmelzpunkt 223-225°.

## Beispiel 23

0,15 g 7-Chlor-5-(2-fluorphenyl)-2-hydrazino-3H-1-benzazepin werden in 10 ml Pyridin gelöst und mit 1,44 ml einer 14,3-proz. Lösung von Phosgen in Toluol versetzt. Das Gemisch wird während 5 Minuten auf 60° erhitzt und dann im Vakuum eingedampft. Der Rückstand wird zwischen Benzol und Wasser verteilt. Die Benzolphase wird nacheinander mit Wasser, Natriumhydrogencarbonatlösung und mit Wasser gewaschen. Das Lösungsmittel wird im Vakuum abgedampft. Der Rückstand wird unter Eluieren mit Benzol/Essigester (19 : 1 und 9 : 1) an 15 g Kieselgel chromatographiert. Nach Umkristallisieren aus Aether/Hexan erhält man reines 8-Chlor-6-(2-fluorphenyl)-2,4-dihydro-1H-s-triazolo [4,3-a] [1]-benzazepin-1-on vom Schmelzpunkt 223-225°.

## Beispiel 24

3 g 7-Chlor-5-(2-fluorphenyl)-2-hydrazino-3H-1-benzazepin werden in 40 ml Dimethylformamid gelöst. Nach Zugabe von 3,6 g N,N'-Carbonyldiimidazol wird das Gemisch während 16 Stunden bei 50° gerührt. Die erhaltene Lösung wird dann auf 700 ml Wasser gegossen und mit Essigester extrahiert. Die organsichen Auszüge werden mit Wasser und gesättigter Natriumchloridlösung gewaschen, getrocknet und eingedampft. Der Rückstand wird unter Eluieren mit Chloroform/Aethanol (98,5 : 1,5) an 200 g Kieselgel chromatographiert. Man erhält nach Umkristallisieren aus Diisopropyläther 8-Chlor-6-(2-fluorphenyl)-2,4-dihydro-1H-s-triazolo [4,3-a] [1] benzazepin-1-on vom Schmelzpunkt 223-225°.

## Beispiel 25

Eine Lösung von 2,65 g 8-Chlor-6-(2-fluorphenyl)-2,4-dihydro-1H-s-triazolo [4,3-a] [1] benzazepin-1-on in einem Gemisch von 16 ml Methanol und 16 ml Methylenchlorid wird bei 0° mit 100 ml ätherischer Diazomethanlösung versetzt. Nach 3 Stunden gibt man nochmals 33 ml Diazomethanlösung dazu und lässt während weiteren 2 Stunden stehen. Nach Eindampfen der Lösung wird der Rückstand unter Eluieren mit Chloroform/Aethanol (99 : 1) an 80 g Kieselgel chromatographiert. Das Rohprodukt wird aus Aether/Hexan umkristallisiert. Man erhält 8-Chlor-6-(2-fluorphenyl)-2,4-dihydro-2-methyl-1H-s-triazolo [4,3-a] [1] benzazepin-1-on vom Schmelzpunkt 142-143°.

## Beispiel 26

5 g 1-Brom-8-chlor-6-(2-fluorphenyl)-4H-s-triazolo-[4,3-a] [1] benzazepin werden in 40 ml N-Methylpiperazin während 21 Stunden unter Rühren auf 120° erhitzt. Nach Abkühlen wird auf Eiswasser gegossen und mit Chloroform extrahiert. Der Rückstand aus den Chloroformauszügen wird an 150 g Kieselgel chromatographiert. Nach Abtrennen von Verunreinigungen durch Eluieren mit Chloroform/Aethanol (94 : 6) erhält man das gewünschte Produkt durch Eluieren mit Chloroform/Aethanol (85 : 15). Nach Umkristallisieren aus Diäthyläther/Diisopropyläther und aus Essigester/Diisopropyläther erhält man 8-Chlor-6-(2-fluorphenyl)-1-(4-methyl-1-piperazinyl)-4H-s-triazolo [4,3-a] [1] benzazepin vom Schmelzpunkt 209-210°.

Beispiel 27

Aus 1-Brom-8-chlor-6-(2-fluorphenyl)-4H-s-triazolo [4,3-a] [1] benzazepin und Morpholin erhält man in Analogie zu Beispiel 26 8-Chlor-6-(2-fluorphenyl)-1-morpholino-4H-s-triazolo [4,3-a] [1] benzazepin vom Schmelzpunkt 246-247°.

Beispiel 28

Aus 1-Brom-8-chlor-6-(2-fluorphenyl)-4H-s-triazolo [4,3-a] [1] benzazepin und Dimethylamin erhält man in Analogie zu Beispiel 26 jedoch unter Verwendung eines Bombenrohres und Erhitzen auf 120° während 2 Stunden und auf 150° während 2 Stunden 1-Dimethylamino-8-chlor-6-(2-fluorphenyl)-4H-s-triazolo [4,3-a] [1] benzazepin vom Schmelzpunkt 240-243°.

Beispiel 29

a) Eine Lösung von 18,2 g 7-Chlor-5-(2-fluorphenyl)-2-hydrazino-3H-1-benzazepin in 220 ml Eisessig wird bei 15° tropfenweise mit 5,1 ml Chloracetylchlorid versetzt. Man rührt noch während 2 Stunden bei Raumtemperatur. Man versetzt mit 7,5 g Natriumacetat, rührt während 2 Stunden bei Raumtemperatur und während 20 Minuten unter Rückfluss zum Sieden, dampft dann in Vakuum ein und verteilt den Rückstand zwischen Chloroform und Wasser. Man wächst die organische Phase mit Natriumhydrogencarbonatlösung und Wasser und dampft ein. Das erhaltene Rohprodukt chromatographiert man an Kieselgel unter Eluieren mit Chloroform/Aethanol (98,5 : 1,5) und erhält 8-Chlor-1-(chlormethyl)-6-(2-fluorphenyl)-4H-s-triazolo [4,3-a] [1] benzazepin als weisse Kristalle vom Schmelzpunkt 180° (Zers.).

b) 7 g 8-Chlor-1-(chlormethyl)-6-(2-fluorphenyl)-4H-s-triazolo [4,3-a] [1] benzazepin und 2,9 g Natriumjodid werden in 195 ml Aceton gelöst. Man rührt während 2 Stunden bei Raumtemperatur, filtriert das ausgefallene Natriumchlorid ab und behandelt das Filtrat unter Rühren bis zur bleibenden basischen Reaktion mit Methylamin. Nach weiteren 10 Minuten wird das Reaktionsgemisch eingedampft. Der Rückstand wird zwischen Essigester und verdünnter Salzsäure verteilt. Die wässerigen Lösungen werden mit Ammoniak alkalisch gestellt, mit Natriumchlorid gesättigt und mit Chloroform extrahiert. Die Chloroformauszüge werden eingedampft. Der Rückstand wird unter Eluieren mit Chloroform/Aethanol (9 : 1) an 300 g Kieselgel chromatographiert. Nach Umkristallisieren aus Essigester/Diisopropyläther erhält man 8-Chlor-6-(2-fluorphenyl)-1-(methylaminomethyl)-4H-s-triazolo [4,3-a] [1] benzazepin vom Schmelzpunkt 170-171°.

Zur Ueberführung in das Hydrochlorid wird dieser Stoff in Aethanol gelöst und mit der berechneten Menge Chlorwasserstoff in Essigester versetzt. Nach Zugabe von Aether kristallisiert 8-Chlor-6-(2-fluorphenyl)-1-(methylaminomethyl)-4H-s-triazolo [4,3-a] [1] benzazepin-hydrochlorid vom Schmelzpunkt 240° (Zers.) aus.

Beispiel 30

Aus 8-Chlor-1-(chlormethyl)-6-(2-fluorphenyl)-4H-s-triazolo [4,3-a] [1] benzazepin und N-Methyl-N-propargylamin erhält man in Analogie zu Beispiel 29b 8-Chlor-6-(2-fluorphenyl)-1-(N-methyl-N-propargylaminomethyl)-4H-s-triazolo [4,3-a] [1] benzazepin vom Schmelzpunkt 147° und das entsprechende Hydrochlorid vom Schmelzpunkt 204° (Zers.).

Beispiel 31

a) Man versetzt eine Lösung von 13,4 g 8-Chlor-6-(2-fluorphenyl)-1,3,4,6-tetrahydro-2-oxo-2H-5,1-benzothiazocin-5,5-dioxid in 380 ml Hexamethylphosphorsäuretriamid mit 7,7 g 2,4-Bis-(4-methoxyphenyl)-1,2,3,4-dithiadiphosphetan-2,4-disulfid, erwärmt während 2 Stunden auf 100°, kühlt wieder auf Raumtemperatur ab und giesst das Reaktionsgemisch auf 8,3 l Wasser. Man extrahiert zweimal mit Essigester und wäscht die organischen Auszüge mit Wasser und mit gesättigter Natriumchloridlösung. Die Essigesterlösungen werden eingedampft, und der Rückstand wird an 500 g Kieselgel chromatographiert. Durch Eluieren mit Chloroform, das 0,5% Aethanol enthält, und Kristallisieren aus Aether erhält man 8-Chlor-6-(2-fluorphenyl)-1,3,4,6-tetrahydro-2-thiono-2H-5,1-benzothiazocin-5,5-dioxid vom Schmelzpunkt 226°.

b) Eine Lösung von 0,9 ml Hydrazin-hydrat in 134 ml Tetrahydrofuran wird mit 6,2 g 8-Chlor-6-(2-fluorphenyl)-1,3,4,6-tetrahydro-2-thiono-2H-5,1-benzothiazocin-5,5-dioxid versetzt. Die Lösung wird unter einem Argonstrom während 1,25 Stunden bei Raumtemperatur gerührt und dann im Vakuum eingedampft. Der Rückstand wird aus Diisopropyläther kristallisiert, wobei man 8-Chlor-6-(2-fluorphenyl)-2-hydrazino-3,6-dihydro-4H-5,1-benzothiazocin-5,5-dioxid vom Schmelzpunkt 159° erhält.

c) Zu einer Lösung von 5,9 g 8-Chlor-6-(2-fluorphenyl)-2-hydrazino-3,6-dihydro-4H-5,1-benzothiazocin-5,5-dioxid in 118 ml Dimethylformamid gibt man 5,8 g N,N'-Carbonyldiimidazol und rührt das Gemisch während 18 Stunden bei 52°. Die erhaltene Lösung wird dann auf 1,8 l Wasser gegossen und dreimal mit 1,2 l Essigester extrahiert. Die organischen Auszüge werden mit Wasser und gesättigter Natriumchloridlösung gewaschen, getrocknet und eingedampft. Der Rückstand wird aus Chloroform kristallisiert und aus Essigester umkristalliseirt. Man erhält 9-Chlor-7-(2-fluorphenyl)-4,5-dihydro-7H-s-triazolo [4,3-a] [1,5] benzothiazocin-1(2H)-on-6.6-dioxid vom Schmelzpunkt 292-293°.

d) 591 mg 9-Chlor-7-(2-fluorphenyl)-4,5-dihydro-7H-s-triazolo [4,3-a] [1,5] benzothiazocin-1 (2H)-on-6,6-dioxid werden in einem Gemisch von 6,0 ml Tetrachlorkohlenstoff, 6,0 ml t-Butanol und 0,34 ml Wasser suspendiert und auf 10° abgekühlt. Unter intensivem Rühren versetzt man mit 1,5 g Kalium-t-butylat, wobei die Temperatur mit einem Eisbad bei 30° gehalten wird. Man rührt noch während 30 Minuten bei 20°, giesst das Reaktionsgemisch auf Wasser und extrahiert fünfmal mit Chloroform. Die Auszüge werden mit Wasser und gesättigter Natriumchloridlösung gewaschen, getrocknet und eingedampft. Der Rückstand wird an 20 g Kiesel chromatographiert. Durch Eluieren mit Chloroform, das 2 % Aethanol enthält, und kristallisieren aus Aether erhält man 8-Chlor-6-(2-fluorphenyl)-2,4-dihydro-1H-s-triazolo [4,3-a] [1] benzazepin-1-on vom Schmelzpunkt 222-224°.

Beispiel 32

a) 500 mg 8-Chlor-1-(chlormethyl)-6-(2-fluorphenyl)-4H-s-triazolo [4,3-a] [1] benzazepin und 212 mg Natriumjodid werden in 14 ml Aceton gelöst. Man rührt während 1 Stunde bei Raumtemperatur und filtriert dann das ausgefallene Natriumchlorid ab. Das Filtrat wird bei 40° im Vakuum eindampft. Der Rückstand, der hauptsächlich aus 8-Chlor-1-(jodmethyl)-6-(2-fluorphenyl)-4H-s-triazolo [4,3-a] [1]-benzazepin besteht, und 308 mg Phthalimidkalium werden in 15 ml Tetrahydrofuran gelöst. Die Lösung wird während 3,5 Tagen bei Raumtemperatur gerührt, dann auf Wasser gegossen und zweimal mit Essigester extrahiert. Die organischen Auszüge werden mit Wasser gewaschen, getrocknet und eingedampft. Der Rückstand wird an 30 g Kieselgel unter Eluieren mit Essigester/Aethanol (9 : 1) chromatographiert und anschliessend aus Essigester/Aether kristallisiert. Man erhält 8-Chlor-1-(phthalimidomethyl)-6-(2-fluorphenyl)-4H-s-triazolo [4,3-a] [1] benzazepin vom Schmelzpunkt 235-237°.

b) 235 mg 8-Chlor-1-(phthalimidomethyl)-6-(2-fluorphenyl)-4H-s-triazolo [4,3-a] [1] benzazepin werden in 15 ml Aethanol gelöst und mit 500 mg Hydrazin-hydrat versetzt. Man rührt während 2 Stunden bei Raumtemperatur und dampft dann das Aethanol im Vakuum ab. Der Rückstand wird in Essigester gelöst, zweimal mit Wasser gewaschen, getrocknet und eingedampft. Der Rückstand wird an 10 g Aluminiumoxyd (neutral, Aktivitätsstufe III) chromatographiert. Durch Eluieren mit Chloroform/Aethanol (9 : 1) und Kristallisieren aus Essigester erhält man 1-(Aminomethyl)-8-chlor-6-(2-fluorphenyl)-4H-s-triazolo [4,3-a] [1] benzazepin vom Schmelzpunkt 187-190°.

Beispiel 33

a) 1,08 g 8-Chlor-1-(chlormethyl)-6-(2-fluorphenyl)-4H-s-triazolo [4,3-a] [1] benzazepin und 458 mg Natriumjodid werden in 30 ml Aceton gelöst. Man rührt während 1 Stunde bei Raumtemperatur, filtriert das ausgefallene Natriumchlorid ab und dampft das Filtrat bei 40° im Vakuum ein. Der Rückstand, der hauptsächlich aus 8-Chlor-1-(jodmethyl)-6-(2-fluorphenyl)-4H-s-triazolo [4,3-a] [1] benzazepin besteht, und 198 mg Natriumazid werden in 30 ml Tetrahydrofuran gelöst. Die Lösung wird während 18 Stunden bei Raumtemperatur gerührt und dann im Vakuum eingedampft. Der Rückstand wird in Chloroform gelöst und zweimal mit Wasser gewaschen. Die organische Phase wird getrocknet, filtriert und dann im Vakuum eingedampft. Der erhaltene Rückstand wird an 20 g Kieselgel unter Eluieren mit Essigester chromatographiert, und liefert nach Kristallisieren aus Isopropyläther 1-(Azidomethyl)-8-chlor-6-(2-fluorphenyl)-4H-s-triazolo [4,3-a] [1] benzazepin vom Schmelzpunkt 147-150°.

b) 734 mg 1-(Azidomethyl)-8-chlor-6-(2-fluorphenyl)-4H-s-triazolo [4,3-a] [1] benzazepin werden in Aethanol gelöst und auf 0° gekühlt. Bei dieser Temperatur wird eine Lösung von 675 mg Zinn (II) chloriddihydrat in 9,5 ml 2N-Natronlauge zugetropft. Das Reaktionsgemisch wird während 45 Minuten bei 0-5° gerührt, dann mit Essigester verdünnt und viermal mit Wasser gewaschen. Die organische Phase wird getrocknet und eingedampft. Durch Kristallisieren aus Essigester erhält man 1-(Aminomethyl)-8-chlor-6-(2-fluorphenyl)-4H-s-triazolo [4,3-a] [1] benzazepin vom Schmelzpunkt 187-190°.

Beispiel 34

a) 30 g 7-Chlor-5-(2-fluorphenyl)-1,3,4,5-tetrahydro-2H-1-benzazepin-2-on werden in 105 ml Hexamethylphosphorsäuretriamid gelöst und mit 22 g 2,4-Bis-(4-methoxyphenyl)-1,2,3,4-dithiadiphosphetan-2,4-disulfid versetzt. Man erwärmt während 1 Stunde auf 100°, kühlt wieder auf Raumtemperatur und giesst das Reaktionsgemisch auf 1,4 l Wasser. Man extrahiert viermal mit Essigester und wäscht die organischen Auszüge mit Wasser. Die Essigesterlösung wird getrocknet, filtriert und im Vakuum eingedampft. Nach Kristallisieren des Rückstandes aus Aethanol erhält man 7-Chlor-5-(2-fluorphenyl)-1,3,4,5-tetrahydro-2H-1-benzazepin-2-thion vom Schmelzpunkt 190°.

b) Eine Lösung von 17,5 ml Hydrazin-hydrat in 1,75 l Tetrahydrofuran wird mit 22 g 7-Chlor-5-(2-fluorphenyl)-1,3,4,5-tetrahydro-2H-1-benzazepin-2-thion versetzt. Die Lösung wird unter einem Argonstrom während 2 Stunden gerührt und dann im Vakuum bei 40° eingedampft. Man kristallisiert den

Rückstand aus Aether und erhält 7-Chlor-5-(2-fluorphenyl)-2-hydrazino-4,5-dihydro-2H-1-benzazepin vom Schmelzpunkt 213-215°.

c) 15,0 g 7-Chlor-5-(2-fluorphenyl)-2-hydrazino-4,5-dihydro-2H-1-benzazepin werden in 200 ml Dimethylformamid gelöst. Nach Zugabe von 18 g N,N'-Carbonyl-diimidazol wird das Gemisch während 20 Stunden bei 52° gerührt, auf 2 l Wasser gegossen und mit Essigester extrahiert. Die organischen Auszüge werden mit Wasser gewaschen, getrocknet und eingedampft, und der Rückstand wird an 800 g Kieselgel chromatographiert. Durch Eluieren mit Chloroform, das 2 % Aethanol enthält, und Kristallisieren aus Aether erhält man 8-Chlor-6-(2-fluorphenyl)-2,4,5,6-tetrahydro-1H-s-triazolo [4,3-a] [1] benzazepin-1-on vom Schmelzpunkt 236-238°.

d) Eine Suspension von 660 mg 8-Chlor-6-(2-fluorphenyl)-2,4,5,6-tetrahydro-1H-s-triazolo [4,3-a] [1] benzazepin-1-on in 150 ml Tetrachlorkohlenstoff wird mit 0,11 ml Brom versetzt und während 2 Stunden unter Bestrahlung mit einer 500 W-Glühlampe und unter Rühren zum Sieden erhitzt. Die Lösung wird im Vakuum eingedampft ; der Rückstand wird in Chloroform gelöst und die Lösung zweimal mit gesättigter Natriumhydrogencarbonat-Lösung gewaschen, getrocknet und im Vakuum eingedampft. Der Rückstand wird an 35 g Kieselgel chromatographiert. Durch Eluieren mit Toluol/Essigester (4 : 1) und Kristallisieren aus Essigester/Aether erhält man 8-Chlor-6-(2-fluorphenyl)-2,4-dihydro-1H-s-triazolo [4,3-a] [1] benzazepin-1-on vom Schmelzpunkt 221-224°.

## Beispiel 35

a) Eine Suspension von 195 mg 6-(2-Chlorphenyl)-1-methyl-8-nitro-4H-s-triazolo [4,3-a] [1] benzazepin in 5 ml konzentrierter Salzsäure wird mit 375 mg Zinn (II) chlorid versetzt, worauf man unter Rühren während 40 Stunden auf 50° erhitzt. Nach Abkühlen auf Raumtemperatur wird das Gemisch mit Natriumhydrogencarbonatlösung neutralisiert und mit Chloroform extrahiert. Die Chloroformauszüge werden getrocknet und dann eingedampft. Der Rückstand wird aus Essigester-Aether kristallisiert, wobei man 8-Amino-6-(2-chlorphenyl)-1-methyl-4H-s-triazolo [4,3-a] [1] benzazepin vom Schmelzpunkt 271-273° erhält.

b) Eine Lösung von 155 mg 8-Amino-6-(2-chlorphenyl)-1-methyl-4H-s-triazolo [4,3-a] [1] benzazepin in 1 ml 2N-Methansulfonsäure wird auf 0° gekühlt und mit einer Lösung von 44 mg Natriumnitrit in 0,2 ml Wasser versetzt. Die Mischung wird 5 Minuten bei 5° gerührt und dann zu einer Lösung von 206 mg Kupfer (I) bromid in 2,4 ml 48 %iger Bromwasserstoffsäure gegeben. Man rührt weitere 10 Minuten bei Raumtemperatur, worauf man mit Natriumhydrogencarbonatlösung neutralisiert und mit Chloroform extrahiert. Die Chloroformauszüge werden getrocknet und dann eingedampft. Der Rückstand wird in Chloroform gelöst und an 5 g Kieselgel chromatographiert. Verunreinigungen werden mit Chloroform und das gewünschte Produkt mit Chloroform/Aethanol (19 : 1) eluiert. Nach Kristallisation aus Essigester-Aether erhält man 8-Brom-6-(2-chlorphenyl)-1-methyl-4H-s-triazolo [4,3-a] [1] benzazepin vom Schmelzpunkt 250-252°.

## Beispiel A

Herstellung von Tabletten nachstehender Zusammensetzung :

| | mg/Tablette |
|---|---|
| 8-Chlor-6-(o-fluorphenyl)-2,4-dihydro-1H-s-triazolo [4,3-a] [1] benzazepin-1-on | 15 |
| Milchzucker | 110 |
| Maisstärke | 61 |
| Talk | 3,4 |
| Magnesiumstearat | 0,5 |
| Tablettengewicht | 189,9 |

Die Bestandteile werden miteinander vermischt und zu Tabletten von je 189,9 mg verpresst. Anschliessend werden sie mit Aethylcellulose und Carbowax überzogen.

Anstelle von 8-Chlor-6-(o-fluorphenyl)-2,4-dihydro-1H-s-triazolo [4,3-a] [1] benzazepin-1-on können auch die folgenden erfindungsgemässen Wirkstoffe in die Tabletten eingearbeitet werden :

8-Chlor-6-(o-fluorphenyl)-1-methyl-4H-s-triazolo [4,3-a] [1] benzazepin,
8-Chlor-6-(o-chlorphenyl)-1-methyl-4H-s-triazolo [4,3-a] [1] benzazepin,
8-Chlor-6-(o-fluorphenyl)-4H-s-triazolo [4,3-a] [1] benzazepin und
1-(Aminomethyl)-8-chlor-6-(o-fluorphenyl)-4H-s-triazolo [4,3-a] [1] benzazepin.

## Beispiel B

Herstellung von Ampullen :

5,0 g 1-(Aminomethyl)-8-chlor-6-(o-fluorphenyl)-4H-s-triazolo [4,3-a] [1] benzazepin-hydrochlorid und

8,5 g Natriumchlorid werden unter leichtem Erwärmen in 900 ml bidestilliertem Wasser gelöst. Man versetzt anschliessend mit 0,1N-Salzsäure, bis ein pH von 4,0-3,5 erreicht wird, und dann mit bidestilliertem Wasser bis zu einem Gesamtvolumen von 1 000 ml. Die Lösung wird in Ampullen von 2 bis 10 ml abgefüllt. Nach Zuschmelzen der Ampullen werden diese auf übliche Weise durch Erhitzen im Autoklaven sterilisiert.

**Patentansprüche** (für die Vertragsstaaten: BE, CH, DE, FR, GB, IT, LI, LU, NL, SE)

1. Benzazepine der allgemeinen Formel

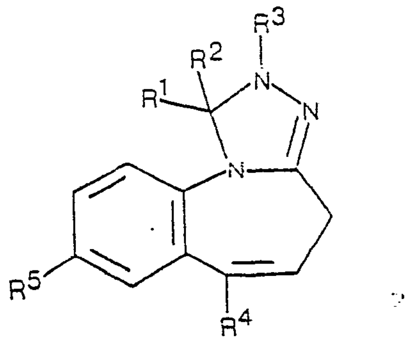

(I)

worin entweder $R^1$ Wasserstoff, niederes Alkyl, 4-Pyridyl oder die Gruppe $-(CH_2)_n-NR^6R^7$ und $R^2$ und $R^3$ zusammen eine zusätzliche Bindung oder $R^1$ und $R^2$ zusammen die Oxogruppe und $R^3$ Wasserstoff oder niederes Alkyl, $R^4$ Phenyl, o-Halogenphenyl oder 2-Pyridyl, $R^5$ Halogen oder Nitro und entweder $R^6$ Wasserstoff oder niederes Alkyl und $R^7$ Wasserstoff, niederes Alkyl, niederes Alkenyl oder niederes Alkinyl oder $R^6$ und $R^7$ zusammen mit dem Stickstoffatom 4-(niederes Alkyl)-1-piperazinyl oder 4-Morpholinyl und n die Zahl 0 oder 1 bedeuten, wobei die als « nieder » bezeichneten Reste höchstens 7 C-Atome enthalten, und pharmazeutisch annehmbare Säureadditionssalze davon.

2. Verbindungen gemäss Anspruch 1, dadurch gekennzeichnet, dass $R^1$ Wasserstoff, Methyl oder Aminomethyl und $R^2$ und $R^3$ zusammen eine zusätzliche Bindung bedeuten oder dass $R^1$ zusammen mit $R^2$ die Oxogruppe und $R^3$ Wasserstoff bedeuten.

3. Verbindungen gemäss Anspruch 1 oder 2, dadurch gekennzeichnet, dass $R^4$ o-Chlorphenyl oder o-Fluorphenyl bedeutet.

4. Verbindungen gemäss einem der Ansprüche 1 bis 3, dadurch gekennzeichnet, dass $R^5$ Chlor bedeutet.

5. 8-Chlor-6-(2-fluorphenyl)-2,4-dihydro-1H-s-triazolo [4,3-a] [1] benzazepin-1-on.

6. 1-(Aminomethyl)-8-chlor-6-(2-fluorphenyl)-4H-s-triazolo [4,3-a] [1] benzazepin.

7. 8-Chlor-6-(2-fluorphenyl)-1-methyl-4H-s-triazolo [4,3-a] [1] benzazepin.

8. Verbindungen der allgemeinen Formeln

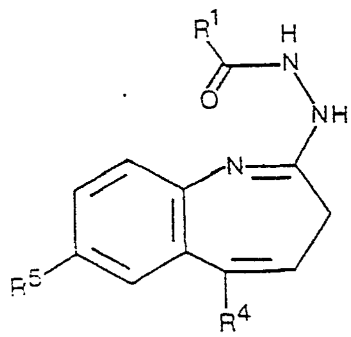

(II)

und

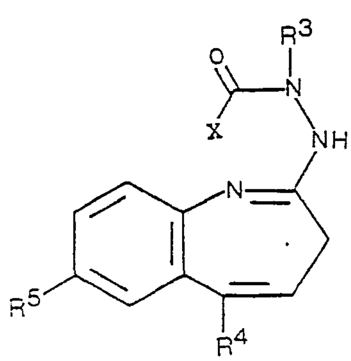

(III)

worin $R^1$, $R^3$, $R^4$ und $R^5$ die in Anspruch 1 angegebene Bedeutung besitzen und X eine Abgangsgruppe bedeutet.

9. Verbindungen der allgemeinen Formel

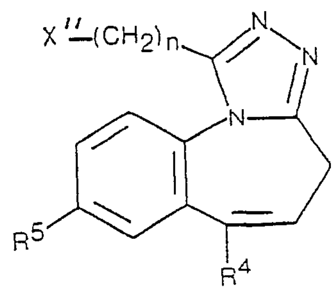

(V)

worin $R^4$, $R^5$ und n die in Anspruch 1 angegebene Bedeutung besitzen und X″ eine Abgangsgruppe bedeutet.

10. Verbindungen der allgemeinen Formel

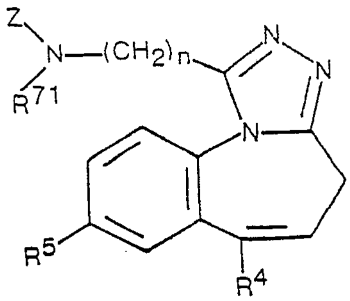

(VII)

worin $R^4$, $R^5$ und n die in Anspruch 1 angegebene Bedeutung besitzen und entweder Z eine Schutzgruppe und $R^{71}$ Wasserstoff, niederes Alkyl, niederes Alkenyl oder niederes Alkinyl oder Z und $R^{71}$ zusammen eine Schutzgruppe bedeuten, wobei die als « nieder » bezeichneten Reste höchstens 7 C-Atome enthalten.

11. Verbindungen der allgemeinen Formel

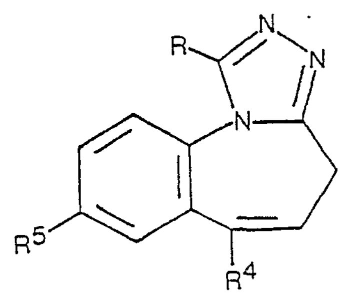

(VIII)

worin R Azido, Azidomethyl, Cyano oder die Gruppe $R^6R^7N$—CO— bedeutet und $R^4$, $R^5$, $R^6$ und $R^7$ die in Anspruch 1 angegebene Bedeutung besitzen.

12. Verbindungen der allgemeinen Formeln

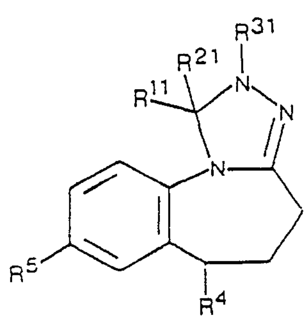

(IX)

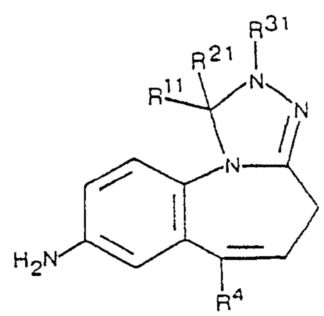

(X)

und

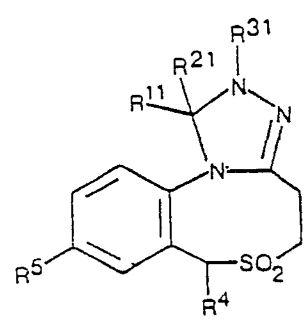

(XI)

worin entweder $R^{11}$ Wasserstoff oder niederes Alkyl und $R^{21}$ und $R^{31}$ zusammen eine zusätzliche Bindung oder $R^{11}$ und $R^{21}$ zusammen die Oxogruppe und $R^{31}$ Wasserstoff oder niederes Alkyl bedeuten und $R^4$ und $R^5$ die in Anspruch 1 angegebene Bedeutung besitzen, wobei die als « nieder » bezeichneten

Reste höchstens 7 C-Atome aufweisen.

13. Verbindungen gemäss einem der Ansprüche 1 bis 7 als pharmazeutische Wirkstoffe.

14. Verbindungen gemäss einem der Ansprüche 1 bis 7 als anxiolytische Wirkstoffe.

15. Verfahren zur Herstellung von Verbindungen gemäss einem der Ansprüche 1 bis 7, dadurch gekennzeichnet, dass man

a) eine Verbindung der allgemeinen Formel

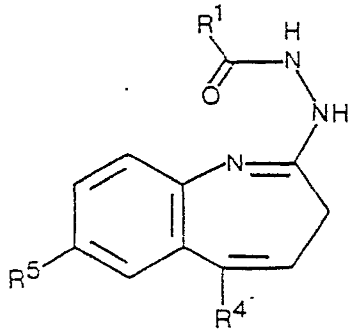

(II) oder

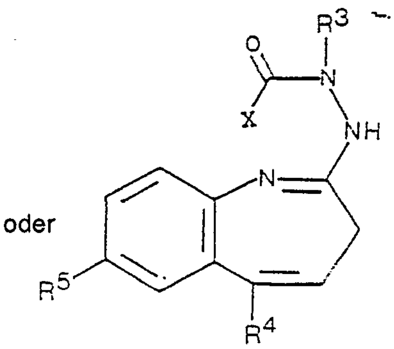

(III)

worin $R^1$, $R^3$, $R^4$ und $R^5$ die in Anspruch 1 angegebene Bedeutung besitzen und X eine Abgangsgruppe bedeutet, cyclisiert, oder

b) eine Verbindung der allgemeinen Formel

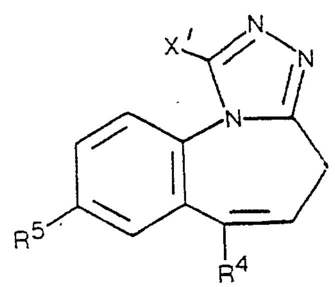

(IV)

worin $R^4$ und $R^5$ die in Anspruch 1 angegebene Bedeutung besitzen und X' eine Abgangsgruppe bedeutet, hydrolysiert, oder

c) eine Verbindung der allgemeinen Formel

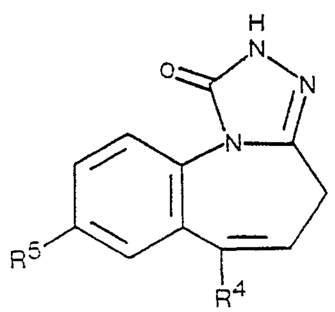

(Ia)

worin $R^4$ und $R^5$ die in Anspruch 1 angegebene Bedeutung besitzen, mit einem einen niederen Alkylrest liefernden Mittel alkyliert, oder

d) eine Verbindung der allgemeinen Formel

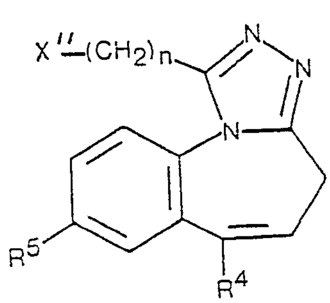

(V)

worin $R^4$, $R^5$ und n die in Anspruch 1 angegebene Bedeutung besitzen und X" eine Abgangsgruppe bedeutet, mit einem Amin der allgemeinen Formel

$$R^6R^7NH \qquad (VI)$$

worin $R^6$ und $R^7$ die in Anspruch 1 angegebene Bedeutung besitzen, umsetzt, oder

e) aus einer Verbindung der allgemeinen Formel

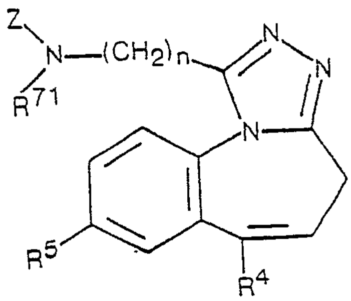

(VII)

worin $R^4$, $R^5$ und n die in Anspruch 1 angegebene Bedeutung besitzen und entweder Z eine Schutzgruppe und $R^{71}$ Wasserstoff, niederes Alkyl, niederes Alkenyl oder niederes Alkinyl oder Z und $R^{71}$ zusammen eine Schutzgruppe bedeuten, die Schutzgruppe abspaltet, oder

f) eine Verbindung der allgemeinen Formel

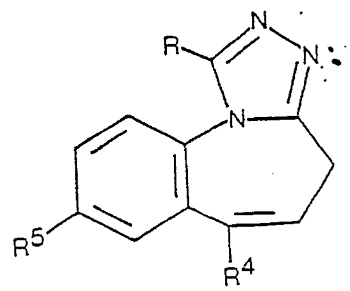

(VIII)

worin R Azido, Azidomethyl, Cyano oder die Gruppe $R^6R^7N$—CO— bedeutet und $R^4$, $R^5$, $R^6$ und $R^7$ die in Anspruch 1 angegebene Bedeutung besitzen, reduziert, oder

g) eine Verbindung der allgemeinen Formel

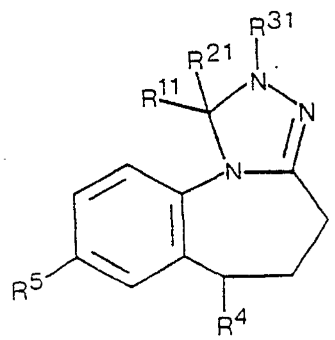

(IX)

worin entweder $R^{11}$ Wasserstoff oder niederes Alkyl und $R^{21}$ und $R^{31}$ zusammen eine zusätzliche Bindung oder $R^{11}$ und $R^{21}$ zusammen die Oxogruppe und $R^{31}$ Wasserstoff oder niederes Alkyl bedeuten und $R^4$ und $R^5$ die in Anspruch 1 angegebene Bedeutung besitzen, dehydriert, oder

h) in einer Verbindung der allgemeinen Formel

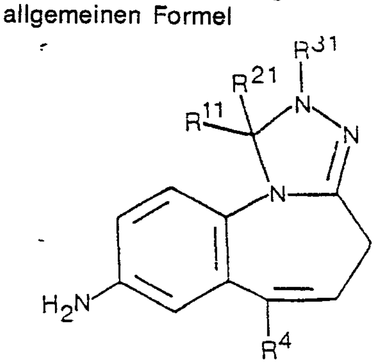

(X)

worin $R^{11}$, $R^{21}$ und $R^{31}$ obige und $R^4$ die in Anspruch 1 angegebene Bedeutung besitzen, die Aminogruppe durch ein Halogenatom oder die Nitrogruppe ersetzt, oder

i) eine Verbindung der allgemeinen Formel

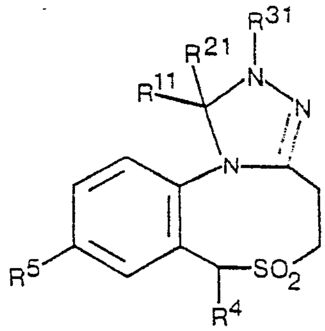

(XI)

worin $R^{11}$, $R^{21}$ und $R^{31}$ obige und $R^4$ und $R^5$ die in Anspruch 1 angegebene Bedeutung besitzen, in Gegenwart einer starken Base mit Tetrachlorkohlenstoff und t-Butanol umsetzt, und

j) erwünschtenfalls eine erhaltene Verbindung der Formel I in ein pharmazeutisch annehmbares Säureadditionssalz überführt.

16. Arzneimittel, enthaltend eine Verbindung gemäss einem der Ansprüche 1 bis 7.

17. Anxiolytika, enthaltend eine Verbindung gemäss einem der Ansprüche 1 bis 7.

**Patentansprüche** (für den Vertragsstaat AT)

1. Verfahren zur Herstellung von Benzazepinen der allgemeinen Formel

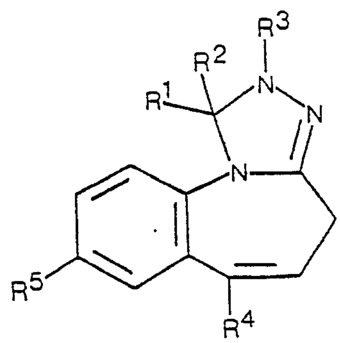

(I)

worin entweder $R^1$ Wasserstoff, niederes Alkyl, 4-Pyridyl oder die Gruppe $-(CH_2)_n-NR^6R^7$ und $R^2$ und $R^3$ zusammen eine zusätzliche Bindung oder $R^1$ und $R^2$ zusammen die Oxogruppe und $R^3$ Wasserstoff oder niederes Alkyl, $R^4$ Phenyl, o-Halogenphenyl oder 2-Pyridyl, $R^5$ Halogen oder Nitro und entweder $R^6$ Wasserstoff oder niederes Alkyl und $R^7$ Wasserstoff, niederes Alkyl, niederes Alkenyl oder niederes Alkinyl oder $R^6$ und $R^7$ zusammen mit dem Stickstoffatom 4-(niederes Alkyl)-1-piperazinyl oder 4-Morpholinyl und n die Zahl 0 oder 1 bedeuten, wobei die als « nieder » bezeichneten Reste höchstens 7 C-Atome aufweisen, und von pharmazeutisch annehmbaren Säureadditionssalzen davon, dadurch gekennzeichnet, dass man

a) eine Verbindung der allgemeinen Formel

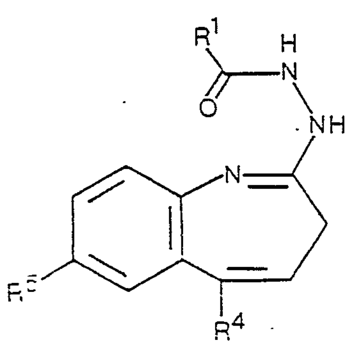

(II) oder

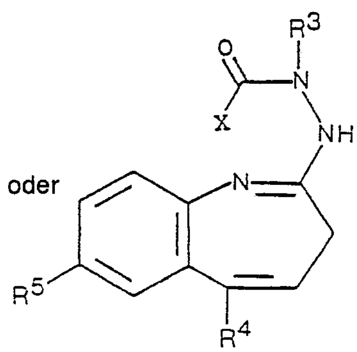

(III)

worin $R^1$, $R^3$, $R^4$ und $R^5$ obige Bedeutung besitzen und X eine Abgangsgruppe bedeutet, cyclisiert, oder

b) eine Verbindung der allgemeinen Formel

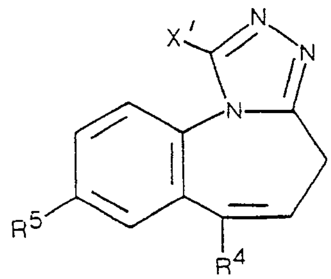

(IV)

worin $R^4$ und $R^5$ obige Bedeutung besitzen und X' eine Abgangsgruppe bedeutet, hydrolysiert, oder

c) eine Verbindung der allgemeinen Formel

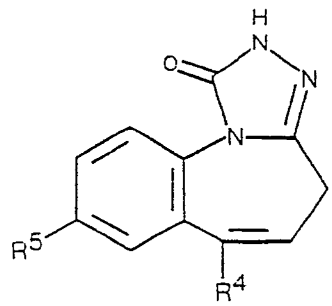

(Ia)

worin $R^4$ und $R^5$ obige Bedeutung besitzen, mit einem einen niederen Alkylrest liefernden Mittel alkyliert, oder

d) eine Verbindung der allgemeinen Formel

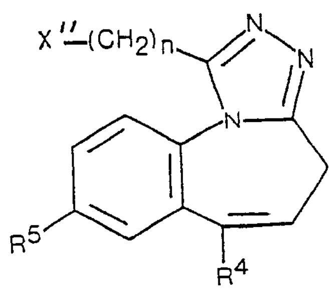

(V)

worin $R^4$, $R^5$ und n obige Bedeutung besitzen und X'' eine Abgangsgruppe bedeutet, mit einem Amin der allgemeinen Formel

$$R^6R^7NH \quad (VI)$$

worin $R^6$ und $R^7$ obige Bedeutung besitzen, umsetzt, oder

e) aus einer Verbindung der allgemeinen Formel

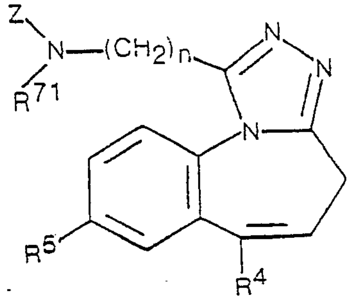

(VII)

worin $R^4$, $R^5$ und n obige Bedeutung besitzen und entweder Z eine Schutzgruppe und $R^{71}$ Wasserstoff, niederes Alkyl, niederes Alkenyl oder niederes Alkinyl oder Z und $R^{71}$ zusammen eine Schutzgruppe bedeuten, die Schutzgruppe abspaltet, oder

f) eine Verbindung der allgemeinen Formel

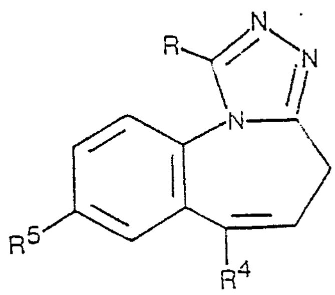

(VIII)

worin R Azido, Azidomethyl, Cyano oder die Gruppe $R^6R^7N—CO—$ bedeutet und $R^4$, $R^5$, $R^6$ und $R^7$ obige Bedeutung besitzen, reduziert, oder

g) eine Verbindung der allgemeinen Formel

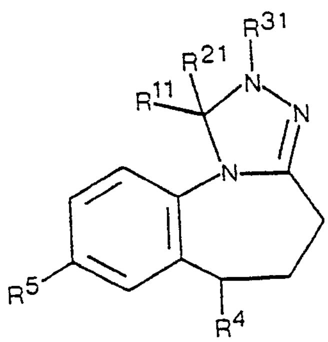

(IX)

worin entweder $R^{11}$ Wasserstoff oder niederes Alkyl und $R^{21}$ und $R^{31}$ zusammen eine zusätzliche Bindung oder $R^{11}$ und $R^{21}$ zusammen die Oxogruppe und $R^{31}$ Wasserstoff oder niederes Alkyl bedeuten und $R^4$ und $R^5$ obige Bedeutung besitzen, dehydriert, oder

h) in einer Verbindung der allgemeinen Formel

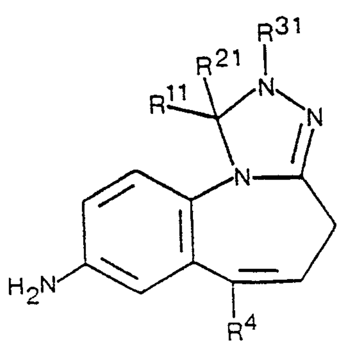

(X)

worin $R^{11}$, $R^{21}$, $R^{31}$ und $R^4$ obige Bedeutung besitzen, die Aminogruppe durch ein Halogenatom oder die Nitrogruppe ersetzt, oder

i) eine Verbindung der allgemeinen Formel

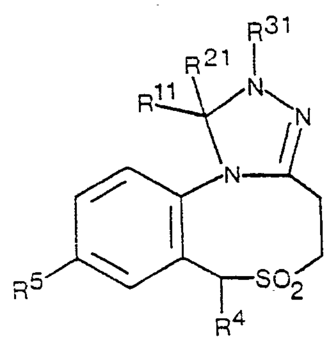

(XI)

worin $R^{11}$, $R^{21}$, $R^{31}$, $R^4$ und $R^5$ obige Bedeutung besitzen, in Gegenwart einer starken Base mit Tetrachlorkohlenstoff und t-Butanol umsetzt, und

j) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass $R^1$ Wasserstoff, Methyl oder Aminomethyl und $R^2$ und $R^3$ zusammen eine zusätzliche Bindung bedeuten oder dass $R^1$ zusammen mit $R^2$ die Oxogruppe und $R^3$ Wasserstoff bedeuten.

3. Verfahren gemäss Anspruch 1 oder 2, dadurch gekennzeichnet, dass $R^4$ o-Chlorphenyl oder o-Fluorphenyl bedeutet.

4. Verfahren gemäss einem der Ansprüche 1 bis 3, dadurch gekennzeichnet, dass $R^5$ Chlor bedeutet.

5. Verfahren gemäss Anspruch 1, dadurch gekennzeichnet, dass man 8-Chlor-6-(2-fluorphenyl)-2,4-dihydro-1H-s-triazolo [4,3-a] [1] benzazepin-1-on herstellt.

6. Verfahren gemäss Anspruch 1, dadurch gekennzeichnet, dass man 1-(Aminomethyl)-8-chlor-6-(2-fluorphenyl)-4H-s-triazolo [4,3-a] [1] benzazepin herstellt.

7. Verfahren gemäss Anspruch 1, dadurch gekennzeichnet, dass man 8-Chlor-6-(2-fluorphenyl)-1-methyl-4H-s-triazolo [4,3-a] [1] benzazepin herstellt.

**Claims** (for the Contracting States: BE, CH, DE, FR, GB, IT, LI, LU, NL, SE)

1. Benzazepines of the general formula

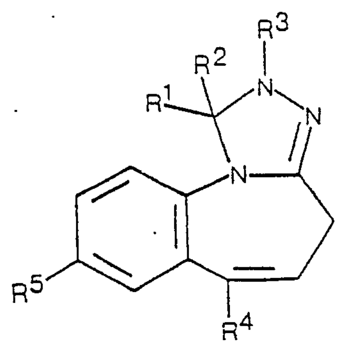

(I)

wherein either $R^1$ signifies hydrogen, lower alkyl, 4-pyridyl or the group $-(CH_2)_n-NR^6R^7$ and $R^2$ and $R^3$ together signify an additional bond or $R^1$ and $R^2$ together signify the oxo group and $R^3$ signifies hydrogen or lower alkyl, $R^4$ signifies phenyl, o-halophenyl or 2-pyridyl, $R^5$ signifies halogen or nitro and either $R^6$ signifies hydrogen or lower alkyl and $R^7$ signifies hydrogen, lower alkyl, lower alkenyl or lower alkynyl or $R^6$ and $R^7$ together with the nitrogen atom signify 4-(lower alkyl)-1-piperazinyl or 4-morpholinyl and n signifies the number 0 or 1, whereby the residues denoted as « lower » contain a maximum of 7 C-atoms, and pharmaceutically acceptable acid addition salts thereof.

2. Compounds in accordance with claim 1, characterized in that $R^1$ signifies hydrogen, methyl or aminomethyl and $R^2$ and $R^3$ together signify an additional bond or in that $R^1$ and $R^2$ together signify the oxo group and $R^3$ signifies hydrogen.

3. Compounds in accordance with claim 1 or 2, characterized in that $R^4$ signifies o-chlorophenyl or o-fluorophenyl.

4. Compounds in accordance with any one of claims 1 to 3, characterized in that $R^5$ signifies chlorine.

5. 8-Chloro-6-(2-fluorophenyl)-2,4-dihydro-1H-s-triazolo [4,3-a] [1] benzazepin-1-one.

6. 1-(Aminomethyl)-8-chloro-6-(2-fluorophenyl)-4H-s-triazolo [4,3-a] [1] benzazepine.

7. 8-Chloro-6-(2-fluorophenyl)-1-methyl-4H-s-triazolo [4,3-a] [1] benzazepine.

8. Compounds of the general formulae

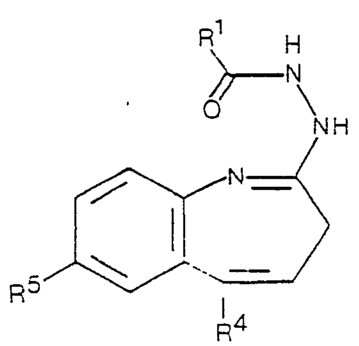

(II)

and

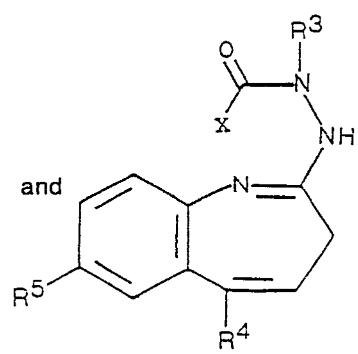

(III)

wherein $R^1$, $R^3$, $R^4$ and $R^5$ have the significance given in claim 1 and X signifies a leaving group.

9. Compounds of the general formula

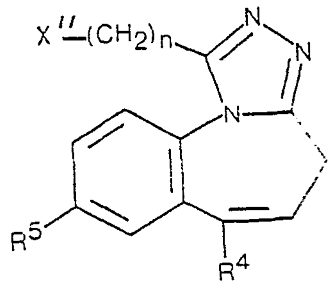

(V)

wherein $R^4$, $R^5$ and n have the significance given in claim 1 and X″ signifies a leaving group.

10. Compounds of the general formula

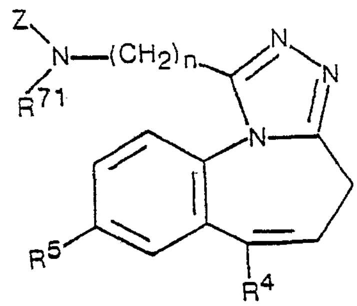

(VII)

wherein $R^4$, $R^5$ and n have the significance given in claim 1 and either Z signifies a protecting group and $R^{71}$ signifies hydrogen, lower alkyl, lower alkenyl or lower alkynyl or Z and $R^{71}$ together signify a protecting group, whereby the residues denoted as « lower » contain a maximum of 7 C-atoms.

11. Compounds of the general formula

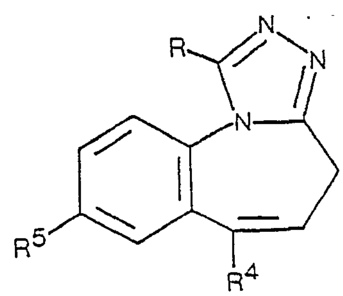

(VIII)

wherein R signifies azido, azidomethyl, cyano or the group $R^6R^7N—CO—$ and $R^4$, $R^5$, $R^6$ and $R^7$ have the significance given in claim 1.

12. Compounds of the general formulae

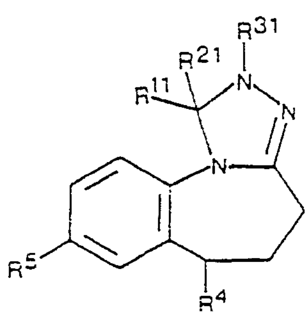

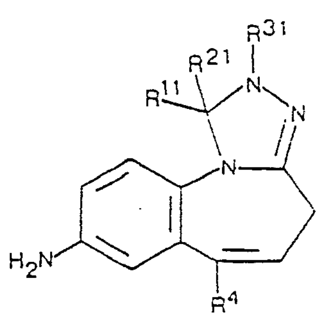

and

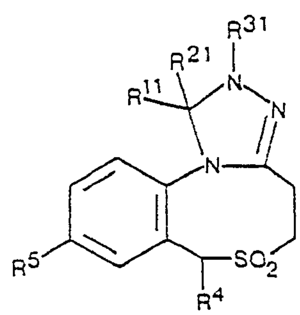

(IX) (X) (XI)

wherein either $R^{11}$ signifies hydrogen or lower alkyl and $R^{21}$ and $R^{31}$ together signify an additional bond or $R^{11}$ and $R^{21}$ together signify the oxo group and $R^{31}$ signifies hydrogen or lower alkyl and $R^4$ and $R^5$ have the significance given in claim 1, whereby the residues denoted as « lower » have a maximum of 7 C-atoms.

13. Compounds in accordance with any one of claims 1 to 7 as pharmaceutically active substances.

14. Compounds in accordance with any one of claims 1 to 7 as anxiolytically active substances.

15. A process for the manufacture of compounds in accordance with any one of claims 1 to 7, characterized by

a) cyclizing a compound of the general formula

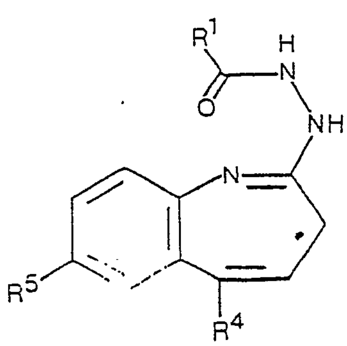

(II)

or

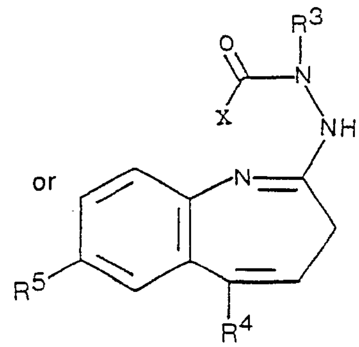

(III)

wherein $R^1$, $R^3$, $R^4$ and $R^5$ have the significance given in claim 1 and X signifies a leaving group, or

b) hydrolyzing a compound of the general formula

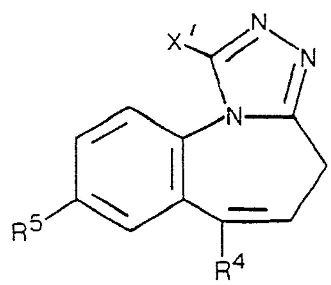

(IV)

wherein $R^4$ and $R^5$ have the significance given in claim 1 and X' signifies a leaving group, or

c) alkylating a compound of the general formula

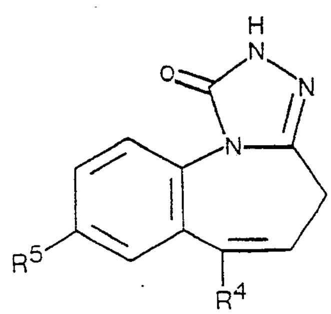

(Ia)

wherein $R^4$ and $R^5$ have the significance given in claim 1, with an agent yielding a lower alkyl residue, or

d) reacting a compound of the general formula

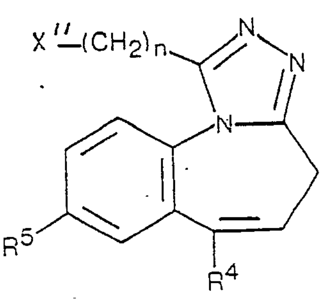

(V)

wherein $R^4$, $R^5$ and n have the significance given in claim 1 and X″ signifies a leaving group, with an amine of the general formula

$$R^6R^7NH \quad (VI)$$

wherein $R^6$ and $R^7$ have the significance given in claim 1, or

e) cleaving off the protecting group from a compound of the general formula

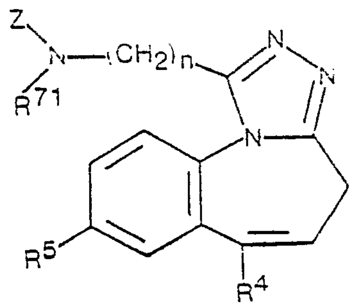

(VII)

wherein $R^4$, $R^5$ and n have the significance given in claim 1 and either Z signifies a protecting group and $R^{71}$ signifies hydrogen, lower alkyl, lower alkenyl or lower alkynyl or Z and $R^{71}$ together signify a protecting group, or

f) reducing a compound of the general formula

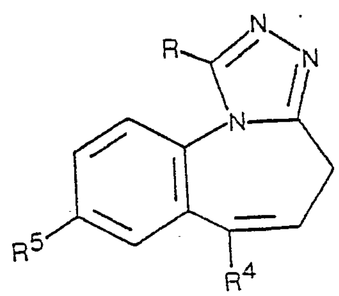

(VIII)

wherein R signifies azido, azidomethyl, cyano or the group $R^6R^7N$—CO— and $R^4$, $R^5$, $R^6$ and $R^7$ have the significance given in claim 1, or

g) dehydrogenating a compound of the general formula

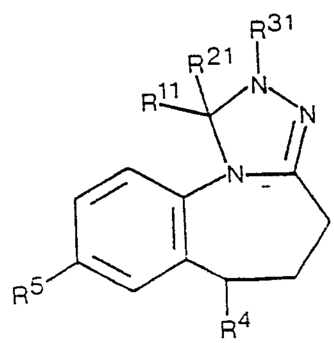

(IX)

wherein either $R^{11}$ signifies hydrogen or lower alkyl and $R^{21}$ and $R^{31}$ together signify an additional bond or $R^{11}$ and $R^{21}$ together signify the oxo group and $R^{31}$ signifies hydrogen or lower alkyl and $R^4$ and $R^5$ have the significance given in claim 1, or

h) replacing the amino group in a compound of the general formula

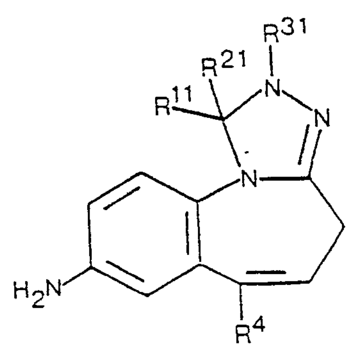

(X)

wherein $R^{11}$, $R^{21}$ and $R^{31}$ have the above significance and $R^4$ has the significance given in claim 1, by a halogen atom or the nitro group, or

i) reacting a compound of the general formula

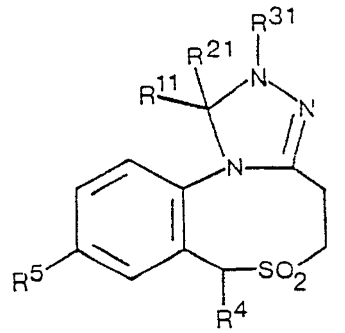

(XI)

wherein $R^{11}$, $R^{21}$ and $R^{31}$ have the above significance and $R^4$ and $R^5$ have the significance given in claim 1, in the presence of a strong base with carbon tetrachloride and t-butanol, and

j) if desired, converting a compound of formula I obtained into a pharmaceutically acceptable acid addition salt.

16. A medicament containing a compound in accordance with any one of claims 1 to 7.

17. An anxiolytic containing a compoud in accordance with any one of claims 1 to 7.

**Claims** (for the Contracting State AT)

1. A process for the manufacture of benzazepines of the general formula

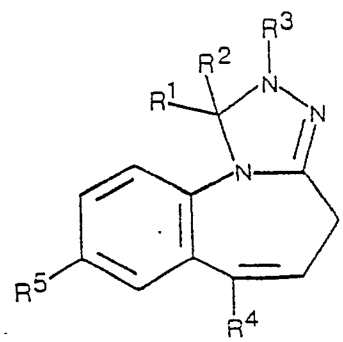

(I)

wherein either $R^1$ signifies hydrogen, lower alkyl, 4-pyridyl or the group $-(CH_2)_n-NR^6R^7$ and $R^2$ and $R^3$ together signify an additional bond or $R^1$ and $R^2$ together signify the oxo group and $R^3$ signifies hydrogen or lower alkyl, $R^4$ signifies phenyl, o-halophenyl or 2-pyridyl, $R^5$ signifies halogen or nitro and either $R^6$ signifies hydrogen of lower alkyl and $R^7$ signifies hydrogen, lower alkyl, lower alkenyl or lower alkynyl or $R^6$ and $R^7$ together with the nitrogen atom signify 4-(lower alkyl)-1-piperazinyl or 4-morpholinyl and n signifies the number 0 or 1, whereby the residues denoted as « lower » have a maximum of 7 C-atoms, and of pharmaceutically acceptable acid addition salts thereof, characterized by

a) cyclizing a compound of the general formula

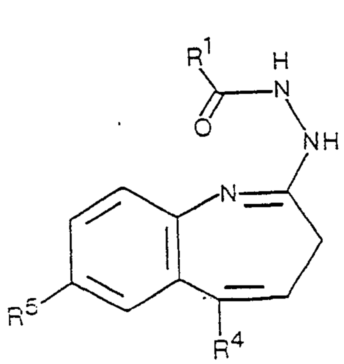

(II) or

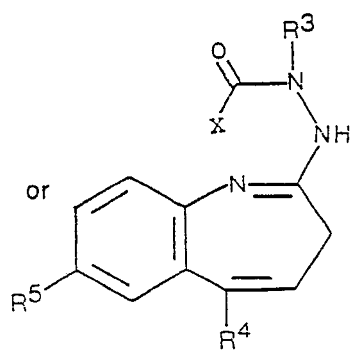

(III)

wherein $R^1$, $R^3$, $R^4$ and $R^5$ have the above significance and X signifies a leaving group, or

b) hydrolyzing a compound of the general formula

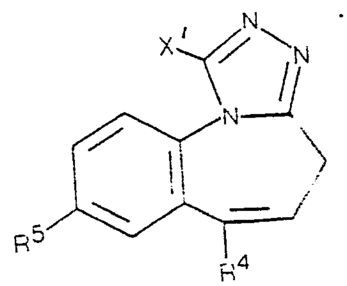

(IV)

wherein $R^4$ and $R^5$ have the above significance and X' signifies a leaving group, or

c) alkylating a compound of the general formula

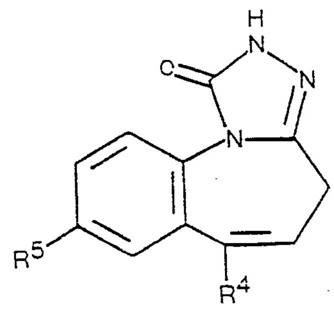

(Ia)

wherein $R^4$ and $R^5$ have the above significance, with an agent yielding a lower alkyl residue, or

d) reacting a compound of the general formula

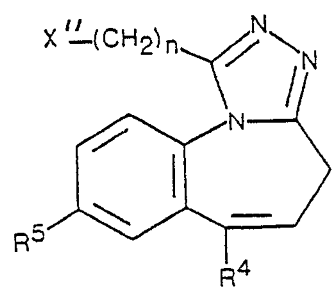

(V)

wherein $R^4$, $R^5$ and n have the above significance and X" signifies a leaving group, with an amine of the general formula

$$R^6R^7NH \quad (VI)$$

wherein $R^6$ and $R^7$ have the above significance, or

e) cleaving off the protecting group from a compound of the general formula

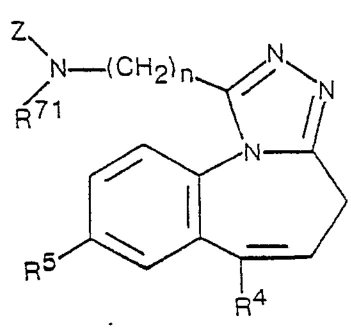

(VII)

wherein $R^4$, $R^5$ and n have the above significance and either Z signifies a protecting group and $R^{71}$ signifies hydrogen, lower alkyl, lower alkenyl or lower alkynyl or Z and $R^{71}$ together signify a protecting group, or

f) reducing a compound of the general formula

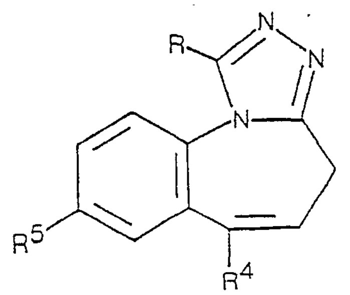

(VIII)

wherein R signifies azido, azidomethyl, cyano or the group $R^6R^7N—CO—$ and $R^4$, $R^5$, $R^6$ and $R^7$ have the above significance, or

g) dehydrogenating a compound of the general formula

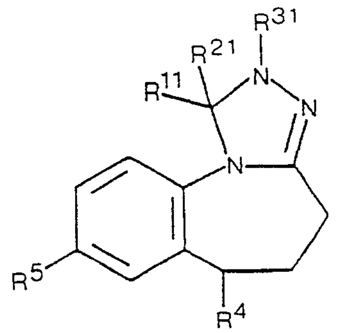

(IX)

wherein either $R^{11}$ signifies hydrogen or lower alkyl and $R^{21}$ and $R^{31}$ together signify an additional bond or $R^{11}$ and $R^{21}$ together signify the oxo group and $R^{31}$ signifies hydrogen or lower alkyl and $R^4$ and $R^5$ have the above significance, or

h) replacing the amino group in a compound of the general formula

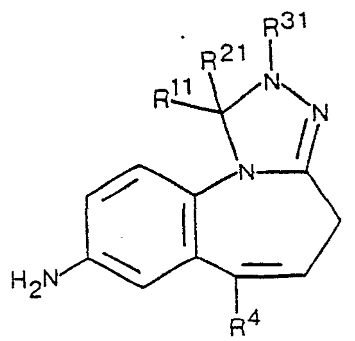

(X)

wherein $R^{11}$, $R^{21}$, $R^{31}$ and $R^4$ have the above significance, by a halogen atom or the nitro group, or

i) reacting a compound of the general formula

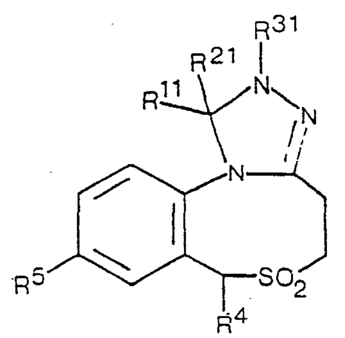

(XI)

wherein $R^{11}$, $R^{21}$, $R^{31}$, $R^4$ and $R^5$ have the above significance, in the presence of a strong base with carbon tetrachloride and t-butanol, and

j) if desired, converting a compound of formula I obtained into a pharmaceutically acceptable acid addition salt.

2. A process in accordance with claim 1, characterized in that $R^1$ signifies hydrogen, methyl or amino-methyl and $R^2$ and $R^3$ together signify an additional bond or in that $R^1$ and $R^2$ together signify the oxo group and $R^3$ signifies hydrogen.

3. A process in accordance with claim 1 or 2, characterized in that $R^4$ signifies o-chlorophenyl or o-fluorophenyl.

4. A process in accordance with any one of claims 1 to 3, characterized in that $R^5$ signifies chlorine.

5. A process in accordance with claim 1, characterized in that 8-chloro-6-(2-fluorophenyl)-2,4-dihydro-1H-s-triazolo [4,3-a] [1] benzazepin-1-one is manufactured.

6. A process in accordance with claim 1, characterized in that 1-(aminomethyl)-8-chloro-6-(2-fluorophenyl-4H-s-triazolo [4,3-a] [1] benzazepine is manufactured.

7. A process in accordance with claim 1, characterized in that 8-chloro-6-(2-fluorophenyl)-1-methyl-4H-s-triazolo [4,3-a] [1] benzazepine is manufactured.

**Revendications** (pour les Etats contractants: BE, CH, DE, FR, GB, LI, LU, NL, SE)

1. Benzazépines de formule générale

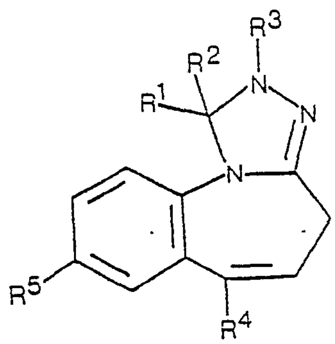

(I)

où ou bien $R^1$ représente un hydrogène, un alcoyle inférieur, un 4-pyridyle ou le groupe $—(CH_2)_n—NR^6R^7$ et $R^2$ et $R^3$ représentent ensemble une liaison supplémentaire, ou bien $R^1$ et $R^2$ représentent ensemble le groupe oxo et $R^3$ représente un hydrogène ou un alcoyle inférieur, $R^4$ représente un phényle, un o-halogènephényle ou un 2-pyridyle, $R^5$ représente un halogène ou un nitro et ou bien $R^6$ représente un hydrogène ou un alcoyle inférieur et $R^7$ représente un hydrogène, un alcoyle inférieur, un alcényle inférieur ou un alcynyle inférieur, ou bien $R^6$ et $R^7$ représentent ensemble avec l'atome d'azote un 4-(alcoyle inférieure)-1-pipérazinyle ou un 4-morpholinyle et n représente le nombre 0 ou 1, où les radicaux décrits comme « inférieurs » contiennent au plus 7 atomes de carbone, et leurs sels d'addition d'acides pharmaceutiquement acceptables.

2. Composés selon la revendication 1, caractérisés en ce que $R^1$ représente un hydrogène, un méthyle ou un aminométhyle et $R^2$ et $R^3$ représentent ensemble une liaison supplémentaire, ou en ce que $R^1$ représente avec $R^2$ le groupe oxo et $R^3$ représente un hydrogène.

3. Composés selon la revendication 1 ou 2, caractérisés en ce que $R^4$ représente un o-chlorophényle ou un o-fluorophényle.

4. Composés selon l'une des revendications 1 à 3, caractérisé en ce que $R^5$ représente un chlore.

5. 8-chloro-6-(2-fluorophényl)-2,4-dihydro-1H-s-triazolo [4,3-a] [1] benzazépin-1-one.

6. 1-(aminométhyl)-8-chloro-6-(2-fluorophényl)-4H-s-triazolo [4,3-a] [1] benzazépine.

7. 8-chloro-6-(2-fluorophényl)-1-méthyl-4H-s-triazolo [4,3-a] [1] benzazépine.

8. Composés de formules générales

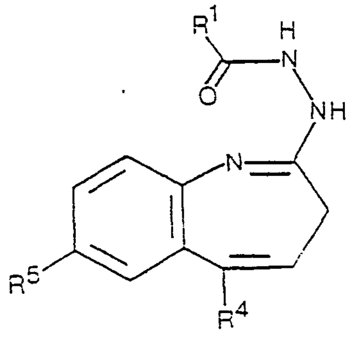

(II)

et

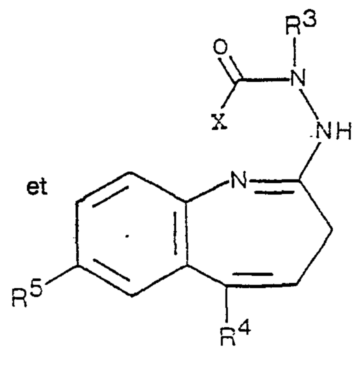

(III)

où $R^1$, $R^3$, $R^4$ et $R^5$ ont la signification donnée dans la revendication 1 et X représente un groupe sortant.

9. Composés de formule générale

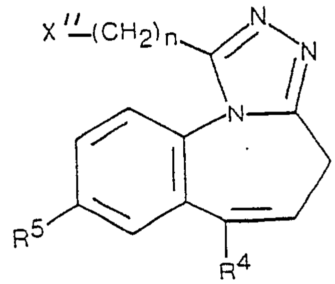

(V)

où $R^4$, $R^5$ et n ont la signification donnée dans la revendication 1 et X″ représente un groupe sortant.

10. Composé de formule générale

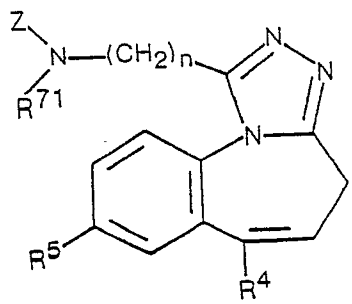

(VII)

où $R^4$, $R^5$ et n ont la signification donnée dans la revendication 1 et ou bien Z représente un groupe protecteur et $R^{71}$ représente un hydrogène, un alcoyle inférieur, un alcényle inférieur ou un alcynyle inférieur, ou bien Z et $R^{71}$ représentent ensemble un groupe protecteur, où les radicaux décrits comme « inférieurs » contiennent au plus 7 atomes de carbone.

11. Composés de formule générale

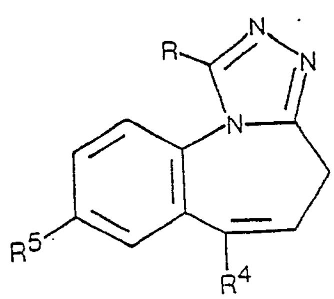

(VIII)

où R représente un azido, un azidométhyle, un cyano ou le groupe $R^6R^7N$—CO— et $R^4$, $R^5$, $R^6$ et $R^7$ ont la signification donnée dans la revendication 1.

12. Composés de formules générales

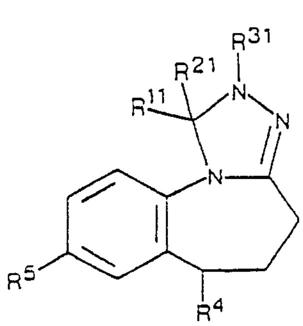

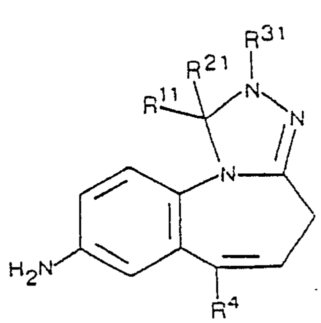

et

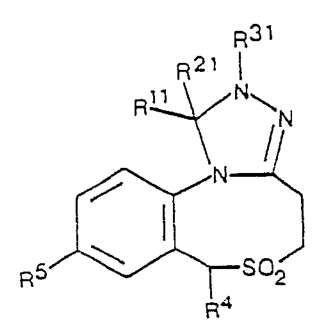

(IX) (X) (XI)

où ou bien $R^{11}$ représente un hydrogène ou un alcoyle inférieur et $R^{21}$ et $R^{31}$ représentent ensemble une liaison supplémentaire, ou bien $R^{11}$ et $R^{21}$ représentent ensemble le groupe oxo et $R^{31}$ représente un hydrogène ou un alcoyle inférieur et $R^4$ et $R^5$ ont la signification donnée dans la revendication 1, où les radicaux décrits comme « inférieurs » présentent au plus 7 atomes de carbone.

13. Composés selon l'une des revendications 1 à 7 comme substances actives pharmaceutiques.

14. Composés selon l'une des revendications 1 à 7 comme substances actives anxiolytiques.

15. Procédé de préparation de composés selon l'une des revendications 1 à 7, caractérisé en ce que

a) on cyclise un composé de formule générale

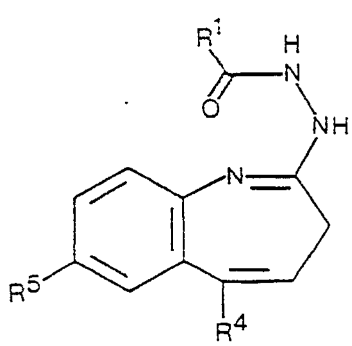 (II) ou 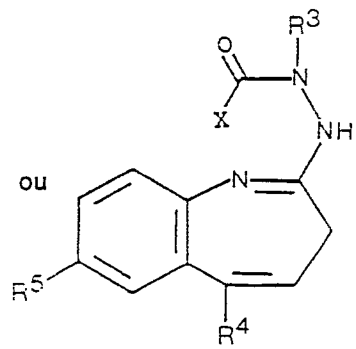 (III)

où $R^1$, $R^3$, $R^4$ et $R^5$ ont la signification donnée dans la revendication 1 et X représente un groupe sortant, ou

b) on hydrolyse un composé de formule générale

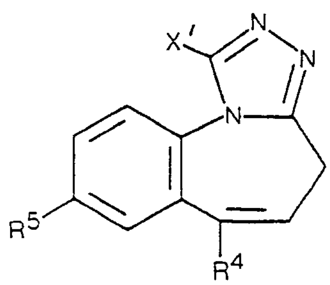 (IV)

où $R^4$ et $R^5$ ont la signification donnée dans la revendication 1 et X' représente un groupe sortant, ou

c) on alcoyle un composé de formule générale

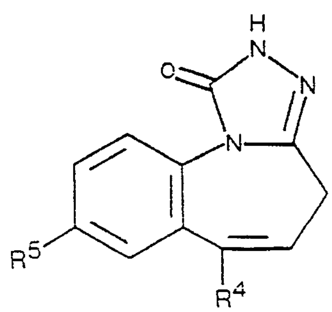 (Ia)

où $R^4$ et $R^5$ ont la signification donnée dans la revendication 1, avec un agent donneur de radical alcoyle inférieur, ou

d) on fait réagir un composé de formule générale

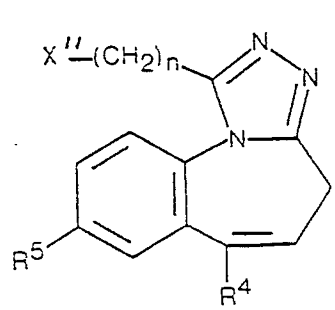 (V)

où $R^4$, $R^5$ et n ont la signification donnée dans la revendication 1 et X″ représente un groupe sortant, avec une amine de formule générale

$$R^6R^7NH \qquad (VI)$$

où $R^6$ et $R^7$ ont la signification donnée dans la revendication 1, ou

e) on sépare le groupe protecteur d'un composé de formule générale

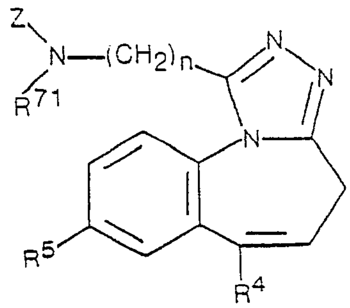

(VII)

où $R^4$, $R^5$ et n ont la signification donnée dans la revendication 1 et ou bien Z représente un groupe protecteur et $R^{71}$ représente un hydrogène, un alcoyle inférieur, un alcényle inférieur ou un alcynyle inférieur ou bien Z et $R^{71}$ représentent ensemble un groupe protecteur, ou

f) on réduit un composé de formule générale

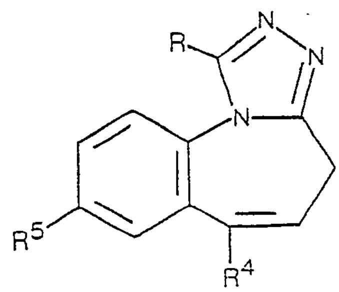

(VIII)

où R représente un azido, un azidométhyle, un cyano ou le groupe $R^6R^7N$—CO— et $R^4$, $R^5$, $R^6$ et $R^7$ ont la signification donnée dans la revendication 1, ou

g) on déshydrogène un composé de formule générale

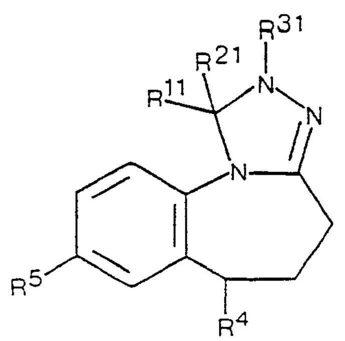

(IX)

où ou bien $R^{11}$ représente un hydrogène ou un alcoyle inférieur et $R^{21}$ et $R^{31}$ représentent ensemble une liaison supplémentaire, ou bien $R^{11}$ et $R^{21}$ représentent ensemble le groupe oxo et $R^{31}$ représente un hydrogène ou un alcoyle inférieur et $R^4$ et $R^5$ ont la signification donnée dans la revendication 1, ou

h) on remplace dans un composé de formule générale

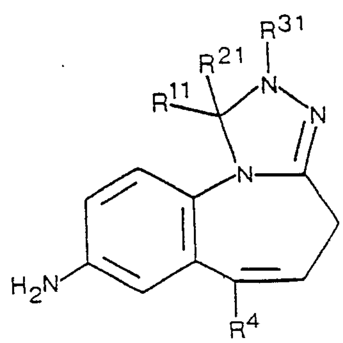

(X)

cu $R^{11}$, $R^{21}$ et $R^{31}$ ont la signification donnée ci-dessus et $R^4$ a la signification indiquée dans la revendication 1, le groupe amino par un atome d'halogene ou le groupe nitro, ou

i) on fait réagir un composé de formule générale

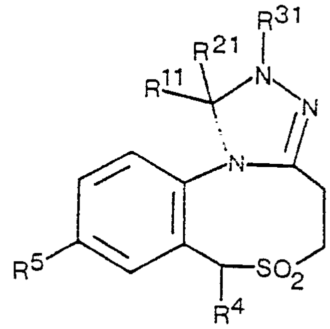

(XI)

où $R^{11}$, $R^{21}$ et $R^{31}$ ont la signification ci-dessus et $R^4$ et $R^5$ ont la signification donnée dans la revendication 1, en présence d'une base forte avec du tétrachlorure de carbone et du t-butanol, et

j) si on le désire on transforme un composé de formule I obtenu en un sel d'addition d'acide pharmaceutiquement acceptable.

16. Médicament contenant un composé selon l'une des revendications 1 à 7.

17. Anxiolytiques contenant un composé selon l'une des revendications 1 à 7.

**Revendications** (pour l'Etat contractant AT)

1. Procédé de préparation de benzazépines de formule générale

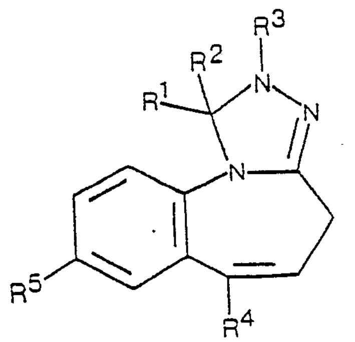

(I)

où ou bien $R^1$ représente un hydrogène, un alcoyle inférieur, un 4-pyridyle ou le groupe $-(CH_2)_n-NR^6R^7$ et $R^2$ et $R^3$ représentent ensemble une liaison supplémentaire, ou bien $R^1$ et $R^2$ représentent ensemble le groupe oxo et $R^3$ représente un hydrogène ou un alcoyle inférieur, $R^4$ représente un phényle, un o-halogènephényle ou un 2-pyridyle, $R^5$ représente un halogène ou un nitro et ou bien $R^6$ représente un hydrogène ou un alcoyle inférieur et $R^7$ représente un hydrogène, un alcoyle inférieur, un alcényle inférieur ou un alcynyle inférieur, ou bien $R^6$ et $R^7$ représentent ensemble avec l'atome d'azote un 4-(alcoyle inférieur)-1-pipérazinyle ou un 4-morpholinyle et n représente le nombre 0 ou 1, où les radicaux décrits comme « inférieurs » contiennent au plus 7 atomes de carbone, et leurs sels d'addition d'acides pharmaceutiquement acceptables, caractérisé en ce que

a) on cyclise un composé de formule générale

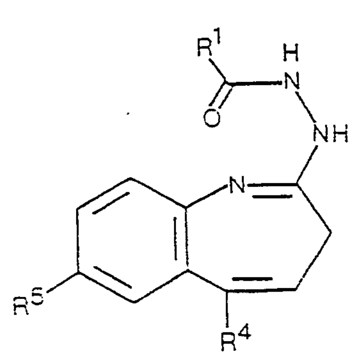

(II)

ou

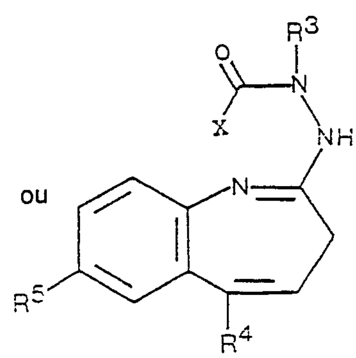

(III)

où $R^1$, $R^3$, $R^4$ et $R^5$ ont la signification donnée ci-dessus et X représente un groupe sortant, ou

b) on hydrolyse un composé de formule générale

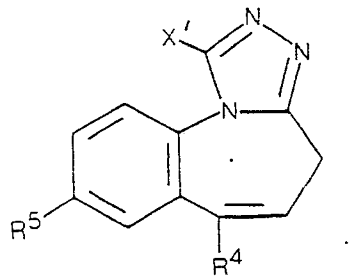

(IV)

où $R^4$ et $R^5$ ont la signification donnée ci-dessus et X' représente un groupe sortant, ou

c) on alcoyle un composé de formule générale

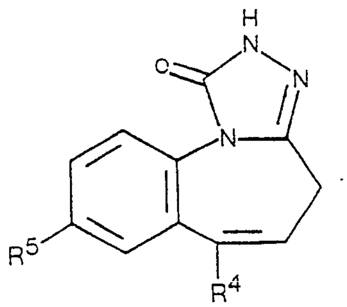

(Ia)

où $R^4$ et $R^5$ ont la signification donnée ci-dessus avec un agent donneur de radical alcoyle inférieur, ou

d) on fait réagir un composé de formule générale

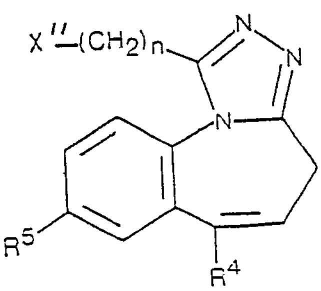

(V)

où $R^4$, $R^5$ et n ont la signification donnée ci-dessus et X" représente un groupe sortant, avec une amine de formule générale

$R^6R^7NH$ (VI)

où $R^6$ et $R^7$ ont la signification donnée ci-dessus, ou

e) on sépare le groupe protecteur d'un composé de formule générale

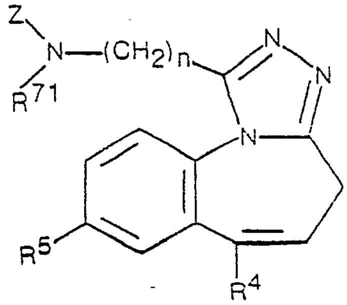

(VII)

où $R^4$, $R^5$ et n ont la signification donnée ci-dessus et ou bien Z représente un groupe protecteur et $R^{71}$ représente un hydrogène, un alcoyle inférieur, un alcényle inférieur ou un alcynyle inférieur, ou bien Z et

$R^{71}$ représentent ensemble un groupe protecteur, ou

f) on réduit un composé de formule générale

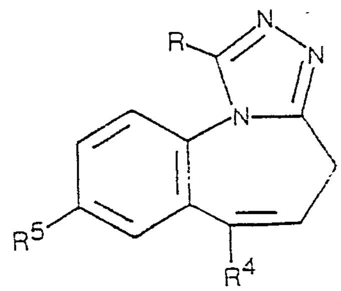

(VIII)

où R représente un azido, un azidométhyle, un cyano ou le groupe $R^6R^7N$—CO— et $R^4$, $R^5$, $R^6$ et $R^7$ ont la signification donnée ci-dessus, ou

g) on déshydrogène un composé de formule générale

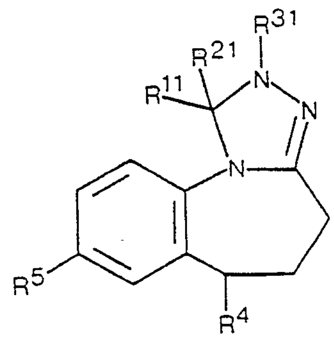

(IX)

où ou bien $R^{11}$ représente un hydrogène ou un alcoyle inférieur et $R^{21}$ et $R^{31}$ représentent ensemble une liaison supplémentaire, ou bien $R^{11}$ et $R^{21}$ représentent ensemble le groupe oxo et $R^{31}$ représente un hydrogène ou un alcoyle inférieur et $R^4$ et $R^5$ ont la signification donnée ci-dessus, ou

h) on remplace dans un composé de formule générale

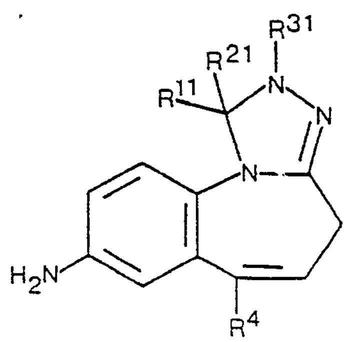

(X)

où $R^{11}$, $R^{21}$, $R^{31}$ et $R^4$ ont la signification donnée ci-dessus le groupe amino par un atome d'halogène ou le groupe nitro, ou

i) on fait réagir un composé de formule générale

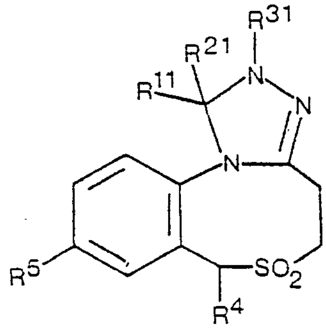

(XI)

où $R^{11}$, $R^{21}$, $R^{31}$, $R^4$ et $R^5$ ont la signification ci-dessus, en présence d'une base forte avec du tétrachlorure de carbone et du t-butanol, et

j) si on le désire on transforme un composé de formule I obtenu en un sel d'addition d'acide pharmaceutiquement acceptable.

2. Procédé selon la revendication 1, caractérisé en ce que $R^1$ représente un hydrogène, un méthyle ou un aminométhyle et $R^2$ et $R^3$ représentent ensemble une liaison supplémentaire ou en ce que $R^1$ représente ensemble avec $R^2$ le groupe oxo et $R^3$ représente un hydrogène.

3. Procédé selon la revendication 1 ou 2, caractérisé en ce que $R^4$ représente un o-chlorophényle ou un o-fluorophényle.

4. Procédé selon l'une des revendications 1 à 3, caractérisé en ce que $R^5$ représente un chlore.

5. Procécé selon la revendication 1, caractérisé en ce qu'on prépare la 8-chloro-6-(2-fluorophényl)-2,4-dihydro-1H-s-triazolo [4,3-a] [1] benzazépin-1-one.

6. Procédé selon la revendication 1, caractérisé en ce qu'on prépare la 1-(aminométhyl)-8-chloro-6-(2-fluorophényl)-4H-s-triazolo [4,3-a] [1] benzazépine.

7. Procédé selon la revendication 1, caractérisé en ce qu'on prépare la 8-chloro-6-(2-fluorophényl)-1-méthyl-4H-s-triazolo [4,3-a] [1] benzazépine.